# EUROPEAN PATENT APPLICATION

(11) **EP 1 604 989 A1**
(43) Date of publication of application: **14.12.2005**
(21) Application number: 04013512.1
(22) Date of filing: 08.06.2004
(51) Int. Cl.: C07D 413/04, C07D 413/14, C07D 403/04, A61K 31/4245, A61K 31/4184, A61P 3/10

(54) **DPP-IV inhibitors**

(71) Applicant: Santhera Pharmaceuticals (Deutschland) Aktiengesellschaft, 69120 Heidelberg (DE)
(72) Inventor: Edwards, Paul John, 69115 Heidelberg (DE); Cerezo-Galvez, Silvia, 42287 Wuppertal (DE)
(74) Representative: Grünecker, Kinkeldey, Stockmair & Schwanhäusser Anwaltssozietät

(57) **Abstract**

The invention relates to compounds of formula (1) 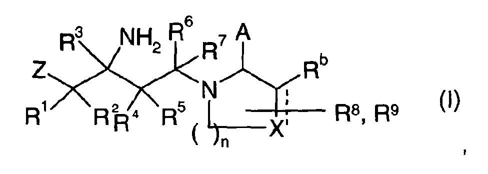 wherein Z, R¹⁻⁹, n, A, X and R^{b} have the meaning as cited in the description and the claims. Said compounds are useful as DPP-IV inhibitors. The invention also relates to the preparation of such compounds as well as the production and use thereof as medicament.

## Description

The present invention relates to a novel class of dipeptidyl peptidase inhibitors, including pharmaceutically acceptable salts and prodrugs thereof, which are useful as therapeutic compounds, particularly in the treatment of Type 2 diabetes mellitus, often referred to as non-insulin dependent diabetes mellitus (NIDDM), and of conditions that are often associated with this disease, such as obesity and lipid disorders.

Diabetes refers to a disease process derived from multiple causative factors and characterized by elevated levels of plasma glucose or hyperglycemia in the fasting state or after administration of glucose during an oral glucose tolerance test. Persistent or uncontrolled hyperglycemia is associated with increased and premature morbidity and mortality. Often abnormal glucose homeostasis is associated both directly and indirectly with alterations of the lipid, lipoprotein and apolipoprotein metabolism and other metabolic and hemodynamic disease. Therefore patients with Type 2 diabetes mellitus are at an increased risk of macrovascular and microvascular complications, including coronary heart disease, stroke, peripheral vascular disease, hypertension, nephropathy, neuropathy, and retinopathy. Therefore, therapeutic control of glucose homeostasis, lipid metabolism and hypertension are critically important in the clinical management and treatment of diabetes mellitus.

There are two generally recognized forms of diabetes. In Type 1, or insulin-dependent, diabetes mellitus (IDDM), patients produce little or no insulin, which is the hormone regulating glucose utilization. In Type 2, or noninsulin dependent, diabetes mellitus (NIDDM), patients often have plasma insulin levels that are the same or elevated compared to nondiabetic subjects. These patients develop a resistance to the insulin stimulating effect on glucose and lipid metabolism in the main insulin-sensitive tissues, namely the muscle, liver and adipose tissues. Further, the plasma insulin levels, while elevated, are insufficient to overcome the pronounced insulin resistance.

Insulin resistance is not primarily due to a diminished number of insulin receptors but to a post-insulin receptor binding defect that is not yet understood. This resistance to insulin responsiveness results in insufficient insulin activation of glucose uptake, oxidation and storage in muscle, and inadequate insulin repression of lipolysis in adipose tissue and of glucose production and secretion in the liver.

The available treatments for Type 2 diabetes, which have not changed substantially in many years, have recognized limitations. While physical exercise and reductions in dietary intake of calories will dramatically improve the diabetic condition, compliance with this treatment is very poor because of well-entrenched sedentary lifestyles and excess food consumption, especially of foods containing high amounts of saturated fat. Increasing the plasma level of insulin by administration of sulfonylureas (e.g., tolbutamide and glipizide) or meglitinide, which stimulate the pancreatic β-cells to secrete more insulin, and/or by injection of insulin when sulfonylureas or meglitinide become ineffective, can result in insulin concentrations high enough to stimulate the very insulin-resistant tissues. However, dangerously low levels of plasma glucose can result from administration of insulin or insulin secretagogues (sulfonylureas or meglitinide), and an increased level of insulin resistance, due to the even higher plasma insulin levels, can occur. The biguanides increase insulin sensitivity resulting in some correction of hyperglycemia. However, the two biguanides, phenformin and metformin, can induce lactic acidosis and nausea/diarrhoea. Metformin has fewer side effects than phenformin and is often prescribed for the treatment of Type 2 diabetes.

The glitazones (*i*.*e*., 5-benzylthiazolidine-2,4-diones) are a recently described class of compounds with potential for ameliorating many symptoms of Type 2 diabetes. These agents substantially increase insulin sensitivity in muscle, liver and adipose tissue in several animal models of Type 2 diabetes, resulting in partial or complete correction of the elevated plasma levels of glucose without occurrence of hypoglycemia. The glitazones that are currently marketed are agonists of the peroxisome proliferator activated receptor (PPAR), primarily the PPAR-gamma subtype. PPAR-gamma agonism is generally believed to be responsible for the improved insulin sensitization that is observed with the glitazones. Newer PPAR agonists that are being tested for treatment of Type 2 diabetes are agonists of the alpha, gamma or delta subtype, or a combination of these, and in many cases are chemically different from the glitazones (i.e., they are not thiazolidinediones). Serious side effects (e.g., liver toxicity) have occurred with some of the glitazones, such as troglitazone.

Additional methods of treating the disease are still under investigation. New biochemical approaches that have been recently introduced or are still under development include treatment with alpha-glucosidase inhibitors (e.g., acarbose) and protein tyrosine phosphatase-IB (PTP-1 B) inhibitors.

Compounds that are inhibitors of the dipeptidyl peptidase-IV (DPP-IV) enzyme are also under investigation as drugs that may be useful in the treatment of diabetes, and particularly Type 2 diabetes. See for example WO-A-97/40832, WO-A-98/19998, WO-A-03/180, WO-A-03/181 and WO-A-2004/007468. The usefulness of DPP-IV inhibitors in the treatment of Type 2 diabetes is based on the fact that DPP-IV *in vivo* readily inactivates glucagon like peptide-1 (GLP-1) and gastric inhibitory peptide (GIP). GLP-1 and GIP are incretins and are produced when food is consumed. The incretins stimulate production of insulin. Inhibition of DPP-IV leads to decreased inactivation of the incretins, and this in turn results in increased effectiveness of the incretins in stimulating production of insulin by the pancreas. DPP-IV inhibition therefore results in an increased level of serum insulin. Advantageously, since the incretins are produced by the body only when food is consumed, DPP-IV inhibition is not expected to increase the level of insulin at inappropriate times, such as between meals, which can lead to excessively low blood sugar (hypoglycemia). Inhibition of DPP-IV is therefore expected to increase insulin without increasing the risk of hypoglycemia, which is a dangerous side effect associated with the use of insulin secretagogues.

DPP-IV inhibitors may also have other therapeutic utilities, as discussed elsewhere in this application. DPP-IV inhibitors have not been studied extensively to date, especially for utilities other than diabetes. New compounds are needed so that improved DPP-IV inhibitors can be found for the treatment of diabetes and potentially other diseases and conditions.

Thus, the object of the present invention is to provide a new class of DPP-IV inhibitors which may be effective in the treatment of Type 2 diabetes and other DPP-IV modulated diseases.

Accordingly, the present invention provides novel compounds of formula (I): 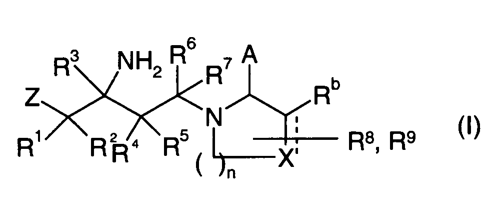 or a pharmaceutically acceptable salt thereof, wherein a dotted line indicates an optionally present double bond and wherein
Z is selected from the group consisting of phenyl; naphthyl; indenyl; C₃₋₇ cycloalkyl; indanyl; tetralinyl; decalinyl; heterocycle; and heterobicycle, wherein Z is optionally substituted with one or more R¹⁰, wherein R¹⁰ is independently selected from the group consisting of halogen; CN; OH; NH₂; oxo (=O), where the ring is at least partially saturated; R¹¹; and R¹²;
R¹¹ is selected from the group consisting of C₁₋₆ alkyl; O-C₁₋₆ alkyl; and S-C₁₋₆ alkyl, wherein R¹¹ is optionally interrupted by oxygen and wherein R¹¹ is optionally substituted with one or more halogen independently selected from the group consisting of F; and Cl;
R¹² is selected from the group consisting of phenyl; heterocycle; and C₃₋₇ cycloalkyl, wherein R¹² is optionally substituted with one or more R¹³, wherein R¹³ is independently selected from the group consisting of halogen; CN; OH; NH₂; oxo (=O), where the ring is at least partially saturated; C₁₋₆ alkyl; O-C₁₋₆ alkyl; and S-C₁₋₆ alkyl;
R¹, R⁴ are independently selected from the group consisting of H; F; OH; and R¹⁴;
R², R⁵, R⁶, R⁷ are independently selected from the group consisting of H; F; and R¹⁵;
R¹⁴ is independently selected from the group consisting of C₁₋₆ alkyl; O-C₁₋₆ alkyl; N(R^{14a})-C₁₋₆ alkyl; S-C₁₋₆ alkyl; C₃₋₇ cycloalkyl; O-C₃₋₇ cycloalkyl; N(R^{14a})-C₃₋₇ cycloalkyl; S-C₃₋₇ cycloalkyl; -C₁₋₆ alkyl-C₃₋₇ cycloalkyl; O-C₁₋₆ alkyl-C₃₋₇ cycloalkyl; N(R^{14a})-C₁₋₆ alkyl-C₃₋₇ cycloalkyl; S-C₁₋₆ alkyl-C₃₋₇ cycloalkyl; heterocycle; O-heterocycle; N(R^{14a})- heterocycle; S-heterocycle; C₁₋₆ alkyl-heterocycle; O-C₁₋₆ alkyl-heterocycle; N(R^{14a})-C₁₋₆ alkyl-heterocycle; S-C₁₋₆ alkyl-heterocycle;
wherein R¹⁴ is optionally substituted with one or more halogen independently selected from the group consisting of F; and Cl;
R^{14a} is selected from the group consisting of H; and C₁₋₆ alkyl;
Optionally R⁶ is selected from the group consisting of -C(R^{6a}R^{6b})-O-C₁₋₆ alkyl; -C(R^{6a}R^{6b})-O-C₃₋₇ cycloalkyl; -C(R^{6a}R^{6b})-S-C₁₋₆ alkyl; -C(R^{6a}R^{6b})-S-C₃₋₇ cycloalkyl; -C(R^{6a}R^{6b})-N(R^{6c})-C₁₋₆ alkyl; and -C(R^{6a}R^{6b})-N(R^{6c})-C₃₋₇ cycloalkyl, wherein each C₁₋₆ alkyl and C₃₋₇ cycloalkyl is optionally substituted with one or more R^{6d}, wherein R^{6d} is independently selected from the group consisting of halogen; C₁₋₆ alkyl; and C₃₋₇ cycloalkyl;
R^{6a}, R^{6b}, R^{6c} are independently selected from the group consisting of H; and C₁₋₆ alkyl;
R¹⁵ is independently selected from the group consisting of C₁₋₆ alkyl; C₃₋₇ cycloalkyl; and -C₁₋₆ alkyl-C₃₋₇ cycloalkyl, wherein R¹⁵ is optionally substituted with one or more R^{15a}, wherein R^{15a} is independently selected from the group consisting of F; Cl; and OH;
R³ is selected from the group consisting of H; and C₁₋₆ alkyl;
Optionally one or more pairs of R¹, R², R³, R⁴, R⁵, R⁶, R^{6a}, R^{6b}, R⁷ independently selected from the group consisting of R¹/R²; R²/R³; R³/R⁴; R⁴/R⁵; R⁵/R⁶; R^{6a}/R^{6b} and R⁶/R⁷ form a C₃₋₇ cycloalkyl ring, which is optionally substituted with one or more of R^{15b}, wherein R^{15b} is independently selected from the group consisting of F; Cl; and OH;
Optionally R⁶, R⁷ jointly form an oxo (=O);
n is 0, 1, 2 or 3;
X is selected from the group consisting of -C(R¹⁶R^{c})-; -C(R^{a})=CR^{c}-; -C(R¹⁶R^{a})-CR^{c}=, -C(R¹⁶R^{a})-O-; -C(R¹⁶R^{a})-S-; -C(R¹⁶R^{a})-S(O)-; -C(R¹⁶R^{a})-S(O)₂-; -C(R¹⁶R^{a})-NR^{c}-; and -C(R¹⁶R^{a})-CR¹⁷R^{c}-;
R⁸ is selected from the group consisting of H; F; OH; and C₁₋₆ alkyl, optionally substituted with one or more halogen selected from the group consisting of F; and Cl;
R⁹, R¹⁶, R¹⁷ are independently selected from the group consisting of H; F; and C₁₋₆ alkyl, optionally substituted with one or more halogen selected from the group consisting of F; and Cl;
R^{a}, R^{c} are independently selected from the group consisting of H; F; Cl; and CN;
R^{b}, A are independently selected from the group consisting of H; F; Cl; CN; and A¹, provided that
(a) one of R^{b}, A is A¹; and
(b) when A is A¹ and n is 1 and R⁶, R⁷ jointly form an oxo (=O), X is other than -C(R¹⁶R^{a})-O-; -C(R¹⁶R^{a})-NR^{c}-; or -C(R¹⁶R^{a})-CR¹⁷R^{c}-; and
(c) when A is A¹ and n is 2 and R⁶, R⁷ jointly form an oxo (=O), X is other than -C(R¹⁶R^{c})-;
Optionally R^{c} is selected from the group consisting of -O-C₁₋₆ alkyl; -O-C₃₋₇ cycloalkyl; -S-C₁₋₆ alkyl; -S-C₃₋₇ cycloalkyl; -N(R¹⁸)-C₁₋₆ alkyl; and -N(R¹⁸)-C₃₋₇ cycloalkyl, wherein each C₁₋₆ alkyl and C₃₋₇ cycloalkyl is optionally substituted with one or more R^{18a}, wherein R^{18a} is independently selected from the group consisting of halogen; C₁₋₆ alkyl; and C₃₋₇ cycloalkyl, provided that n is 1;
R¹⁸ is independently selected from the group consisting of H; C₁₋₆ alkyl;
Optionally a pair of R^{a}, R^{b}, R^{c} selected from the group consisting of R^{a}/R^{c}; and R^{b}/R^{c} forms a ring Z¹; provided that R^{b}/R^{c} form a ring other than
(a) pyrimidine when R⁶, R⁷ jointly form an oxo (=O) and n is 1 and X is -C(R¹⁶R^{a})-C(R^{c})=;
(b) a ring selected from the group consisting of 1,2-diazole; and 1,2,4-triazole when R⁶, R⁷ jointly form an oxo (=O) and n is 1 and X is -C(R¹⁶ R^{a})-N(R^{c})-;
Z¹ is selected from the group consisting of Z²; and Z³;
Z² is selected from the group consisting of phenyl; naphthyl; and indenyl; wherein Z² is optionally substituted with one or more R¹⁹; wherein R¹⁹ is independently selected from the group consisting of halogen; CN; COOR²⁰; OR²⁰; C(O)N(R²⁰R^{20a}); S(O)₂N(R²⁰R^{20a}); C₁₋₆ alkyl; O-C₁₋₆ alkyl; S-C₁₋₆ alkyl; COO-C₁₋₆ alkyl; OC(O)-C₁₋₆ alkyl; C(O)N(R²⁰)-C₁₋₆ alkyl; S(O)₂N(R²⁰)-C₁₋₆ alkyl; S(O)N(R²⁰)-C₁₋₆ alkyl; S(O)₂-C₁₋₆ alkyl; S(O)-C₁₋₆ alkyl; N(R²⁰)S(O)₂-C₁₋₆ alkyl; and N(R²⁰)S(O)-C₁₋₆ alkyl; wherein each C₁₋₆ alkyl is optionally substituted with one more halogen selected from the group consisting of F; and Cl;
Z³ is selected from the group consisting of C₃₋₇ cycloalkyl; indanyl; tetralinyl; decalinyl; heterocycle; and heterobicycle; wherein Z³ is optionally substituted with one or more R²¹, wherein R²¹ is independently selected from the group consisting of halogen; CN; OR²²; oxo (=O), where the ring is at least partially saturated; N(R²²R^{22a}); COOR²²; C(O)N(R²²R^{22a}); S(O)₂N(R²²R^{22a}); S(O)N(R²²R^{22a}); C₁₋₆ alkyl; O-C₁₋₆ alkyl; S-C₁₋₆ alkyl; N(R²²)-C₁₋₆ alkyl; COO-C₁₋₆ alkyl; OC(O)- C₁₋₆ alkyl; C(O)N(R²²)- C₁₋₆ alkyl; N(R²²)-C(O)-C₁₋₆ alkyl; S(O)₂N(R²²)-C₁₋₆ alkyl; S(O)N(R²²)-C₁₋₆ alkyl; S(O)₂-C₁₋₆ alkyl; S(O)-C₁₋₆ alkyl; N(R²²)S(O)₂-C₁₋₆ alkyl; and N(R²²)S(O)-C₁₋₆ alkyl; wherein each C₁₋₆ alkyl is optionally substituted with one more halogen selected from the group consisting of F; and Cl;
Optionally R²¹ is C(O)R²², provided that C(O)R²² is bound to a nitrogen, which is a ring atom of a heterocycle or heterobicycle;
R²⁰, R^{20a}, R²², R^{22a} are independently selected from the group consisting of H; C₁₋₆ alkyl; C₃₋₇ cycloalkyl; and -C₁₋₆ alkyl-C₃₋₇ cycloalkyl;
A¹ is selected from the group consisting of phenyl; heterocycle; heterobicycle; C₃₋₇ cycloalkyl, wherein A¹ is substituted with R²³ and wherein phenyl is optionally substituted with one R²⁴ and wherein heterocycle; heterobicycle; C₃₋₇ cycloalkyl are optionally substituted with one R²⁵;
R²⁴ is selected from the group consisting of halogen; CN; COOR²⁶; OC(O)R²⁶; OR²⁶; -C₁₋₆alkyl-OR²⁶; SR²⁶; C(O)N(R²⁶R²⁷); S(O)₂N(R²⁶R²⁷); S(O)N(R²⁶R²⁷); C₁₋₆ alkyl; N(R²⁶)S(O)₂R²⁷; and N(R²⁶)S(O)R²⁷; wherein each C₁₋₆ alkyl is optionally substituted with one more halogen selected from the group consisting of F; and Cl;
R²⁵ is selected from the group consisting of halogen; CN; OR²⁶; -C₁₋₆alkyl-OR²⁶ SR²⁶; oxo (=O), where the ring is at least partially saturated; N(R²⁶R²⁷); _{COOR}²⁶; OC(O)R²⁶; C(O)N(R²⁶R²⁷); S(O)₂N(R²⁶R²⁷); S(O)N(R²⁶R²⁷); C₁₋₆ alkyl; N(R²⁶)C(O)R²⁷; S(O)₂R²⁶; S(O)R²⁶; N(R²⁶)S(O)₂R²⁷; and N(R²⁶)S(O)R²⁷; wherein each C₁₋₆ alkyl is optionally substituted with one more halogen selected from the group consisting of F; and Cl;
Optionally R²⁵ is C(O)R²⁶, provided that C(O)R²⁶ is bound to a nitrogen, which is a ring atom of a heterocycle or heterobicycle;
R²⁶, R²⁷ are independently selected from the group consisting of H; C₁₋₆ alkyl; C₃₋₇ cycloalkyl; and -C₁₋₆ alkyl-C₃₋₇ cycloalkyl; wherein each C₁₋₆ alkyl is optionally substituted with one more halogen selected from the group consisting of F; and Cl;
R²³ is selected from the group consisting of F; Cl; oxo (=O), where the ring is at least partially saturated; OR^{23a}; N(R^{23a}R^{23b}); C₁₋₆ alkyl, optionally substituted with one or more R²⁸; and -(C(R²⁹R^{29a}))ₘ-W-(C(R³⁰R^{30a}))ₒ-T;
R²⁸ is selected from the group consisting of halogen; CN; COOR³¹; OC(O)R³¹; OR³¹; SR³¹; C(O)N(R³¹R³²); S(O)₂N(R³¹R³²); S(O)N(R³¹R³²); N(R³¹)S(O)₂R³²; and N(R³¹)S(O)R³²;
R²⁹, R^{29a}, R³⁰, R^{30a} are independently selected from the group consisting of H; F; and R³³;
Optionally one or both pairs of R²⁹, R^{29a}, R³⁰, R^{30a} independently selected from the group consisting of R²⁹/R^{29a}; and R³⁰/R^{30a} form a C₃₋₇ cycloalkyl ring, which is optionally substituted with one or more of R^{33b}, wherein R^{33b} is independently selected from the group consisting of F; Cl; and OH;
R^{23a}, R^{23b}, R³¹, R³² are independently selected from the group consisting of H; C₁₋₆ alkyl; C₃₋₇ cycloalkyl; and -C₁₋₆ alkyl-C₃₋₇ cycloalkyl; wherein each C₁₋₆ alkyl is optionally substituted with one more halogen selected from the group consisting of F; and Cl;
R³³ is selected from the group consisting of C₁₋₆ alkyl; C₃₋₇ cycloalkyl; and -C₁₋₆ alkyl-C₃₋₇ cycloalkyl, wherein R³³ is optionally substituted with one or more R^{33a}, wherein R^{33a} is independently selected from the group consisting of F; Cl; and OH;
m, o are independently selected from the group consisting of 0; 1; 2; and 3;
W is selected from the group consisting of a covalent bond; -O-; -S-; -S(O₂)-; -S(O)-; -N(R³⁴)-; -N(R³⁴)C(O)-; -C(O)N(R³⁴)-; -OC(O)-; -C(O)O-; -S(O₂)N(R³⁴)-; -N(R³⁴)S(O)₂-; S(O)N(R³⁴)-; and -N(R³⁴)S(O)-;
R³⁴ is selected from the group consisting of H; C₁₋₆ alkyl; C₃₋₇ cycloalkyl; and -C₁₋₆ alkyl-C₃₋₇ cycloalkyl; wherein each C₁₋₆ alkyl is optionally substituted with one more halogen selected from the group consisting of F; and Cl;
T is selected from the group consisting of H; T¹; and T²;
T¹ is selected from the group consisting of phenyl; naphthyl; and indenyl; wherein T¹ is optionally substituted with one or more R³⁵; wherein R³⁵ is independently selected from the group consisting of halogen; CN; COOR³⁷; OC(O)R³⁷; OR³⁷; -C₁₋₆alkyl-OR³⁷; SR³⁷; S(O)R³⁷; S(O)₂R³⁷; C(O)N(R³⁷R³⁸); S(O)₂N(R³⁷R³⁸); S(O)N(R³⁷R³⁸); C₁₋₆ alkyl; N(R³⁷)S(O)₂R³⁸; and N(R³⁷)S(O)R³⁸; wherein each C₁₋₆ alkyl is optionally substituted with one more halogen selected from the group consisting of F; and Cl;
T² is selected from the group consisting of C₃₋₇ cycloalkyl; indanyl; tetralinyl; decalinyl; heterocycle; and heterobicycle; wherein T² is optionally substituted with one or more R³⁶, wherein R³⁶ is independently selected from the group consisting of halogen; CN; OR³⁷; -C₁₋₆alkyl-OR³⁷ SR³⁷; oxo (=O), where the ring is at least partially saturated; N(R³⁷R³⁸); COOR³⁷; OC(O)R³⁸; C(O)N(R³⁷R³⁸); S(O)₂N(R³⁷R³⁸); S(O)N(R³⁷R³⁸); C₁₋₆ alkyl; N(R³⁷)C(O)R³⁸; S(O)₂R³⁷; S(O)R³⁸; N(R³⁷)S(O)₂R³⁸; and N(R³⁷)S(O)R³⁸; wherein each C₁₋₆ alkyl is optionally substituted with one more halogen selected from the group consisting of F; and Cl;
Optionally R³⁶ is C(O)R³⁷, provided that C(O)R³⁷ is bound to a nitrogen, which is a ring atom of a heterocycle or heterobicycle;
R³⁷, R³⁸ are independently selected from the group consisting of H; C₁₋₆ alkyl; C₃₋₇ cycloalkyl; and -C₁₋₆ alkyl-C₃₋₇ cycloalkyl; wherein each C₁₋₆ alkyl is optionally substituted with one more halogen selected from the group consisting of F; and Cl.

Within the meaning of the present invention the terms are used as follows:

In case a variable or substituent can be selected from a group of different variants and such variable or substituent occurs more than once the respective variants can be the same or different.

"Alkyl" means a straight-chain or branched carbon chain that may contain double or triple bonds. It is generally preferred that alkyl doesn't contain double or triple bonds. "C₁₋₄ Alkyl" means an alkyl chain having 1 - 4 carbon atoms, e.g. at the end of a molecule methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl or amid, e.g. -CH₂-, -CH₂-CH₂-, -CH=CH-, -CH(CH₃)-, -C(CH₂)-, -CH₂-CH₂-CH₂-, -CH(C₂H₅)-, -CH(CH₃)₂-.
"C₁₋₆ Alkyl" means an alkyl chain having 1 - 6 carbon atoms, e.g. C₁₋₄ alkyl, methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl, n-pentane, n-hexane, or amid, e.g. -CH₂-, -CH₂-CH₂-, -CH=CH-, -CH(CH₃)-, -C(CH₂)-, -CH₂-CH₂-CH₂-, -CH(C₂H₅)-, -CH(CH₃)₂-. Each hydrogen of a C₁₋₆ alkyl carbon may be replaced by a substituent.

"C₃₋₇ Cycloalkyl" or "C₃₋₇ Cycloalkyl ring" means a cyclic alkyl chain having 3 - 7 carbon atoms, e.g. cyclopropyl, cyclobutyl, cyclopentyl, cyclohexyl, cyclohexenyl, cycloheptyl. Each hydrogen of a cycloalkyl carbon may be replaced by a substituent.

"Halogen" means fluoro, chloro, bromo or iodo. It is generally preferred that halogen is fluoro or chloro.

"Heterocycle" means a cyclopentane, cyclohexane or cycloheptane ring that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine. "Heterocycle" means also azetidine.

"Heterobicycle" means a heterocycle which is condensed with phenyl or an additional heterocycle to form a bicyclic ring system. "Condensed" to form a bicyclic ring means that two rings are attached to each other by sharing two ring atoms. Examples for a heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, dihydroquinoline, isoquinoline, tetrahydroisoquinoline, dihydroisoquinoline, benzazepine, purine or pteridine.

A preferred stereochemistry of compounds or a pharmaceutically acceptable salt thereof according to the present invention is shown in formula (Ia): 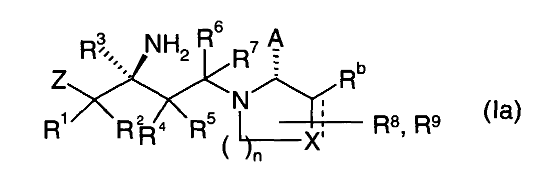 or a pharmaceutically acceptable salt thereof, wherein Z, R¹⁻⁹, n, A, X and R^{b} have the meaning as indicated above.

Preferred compounds of formula (I) or (Ia) are those compounds in which one or more of the residues contained therein have the meanings given below, with all combinations of preferred substituent definitions being a subject of the present invention. With respect to all preferred compounds of the formulas (I) or (Ia) the present invention also includes all tautomeric and stereoisomeric forms and mixtures thereof in all ratios, and their pharmaceutically acceptable salts.

In preferred embodiments of the present invention, the Z, R¹⁻⁹, n, A, X and R^{b} of the formula (I) or (Ia) independently have the following meaning. Hence, one or more of the substituents Z, R¹⁻⁹, n, A, X and R^{b} can have the preferred or more preferred meanings given below.

Preferably, Z is selected from the group consisting of phenyl; and heterocycle; and wherein Z is optionally substituted with up to 2 R¹⁰, which are the same or different.

Preferably, R¹⁰ is selected from the group consisting of F; Cl; CN; and C₁₋₆ alkyl.

Preferably, R¹, R², R⁴, R⁵ are independently selected from the group consisting of H; F; and C₁₋₆ alkyl, optionally substituted with one or more F.

Preferably, R³ is H.

Preferably, R⁶ and R⁷ jointly form an oxo (=O) or R⁶ and R⁷ are independently selected from the group consisting of H; and C₁₋₆ alkyl, optionally substituted with one or more F.

Preferably, n is 0 and X is -CHR^{a}-CHR^{b}-;

Preferably, n is 1 and X is -CHR^{c}-.

Preferably, n is 1 and X is -C(R^{a})=CR^{c}- and R^{a}/R^{c} forms a ring Z¹.

Preferably, n is 1 and X is -CH(R^{a})-CR^{c}= and R^{a}/R^{c} forms a phenyl.

Preferably, Z¹ is phenyl.

Preferably, R⁸, R⁹ are H.

Preferably, A is A¹.

Preferably, R^{a}, R^{b}, R^{c} are H.

Preferably, A¹ is heterocycle.

More preferred, A¹ is selected from the group consisting of 1,2,4-oxadiazole; 1,2,4-triazole; 1,2,3-triazole; 1,2-diazole; and benzimidazole; wherein A¹ is substituted with R²³ and optionally substituted with R²⁵.

Preferably, R²³ is -(C(R²⁹R^{29a}))ₘ-W-(C(R³⁰R^{30a}))ₒ-T.

Preferably, R²⁵ is Cl.

Preferably, m and o are 0.

Preferably, W is a covalent bond.

Preferably, T is H; or T is phenyl, optionally substituted with one or two R³⁵, which are the same or different; or T is selected from the group consisting of heterocycle; and C₃₋₇ cycloalkyl; wherein T is optionally substituted with one or two R³⁶, which are the same or different.

More preferred, T is selected from the group consisting of pyridine; azetidine; cyclopropyl; and cyclobutyl.

Preferably, R³⁵, R³⁶ are independently selected from the group consisting of F; Cl; -S(O)₂-C₁₋₆alkyl; -S(O)₂NH₂; -S(O)₂(C₁₋₆ alkyl)₂; -NH-S(O)₂-C₁₋₆ alkyl; and -N(C₁₋₆ alkyl)-S(O)₂-C₁₋₆ alkyl.

Compounds of the formula (I) or (Ia) in which some or all of the above-mentioned groups have the preferred or more preferred meanings are also an object of the present invention.

Preferred embodiments of the compounds according to present invention are:

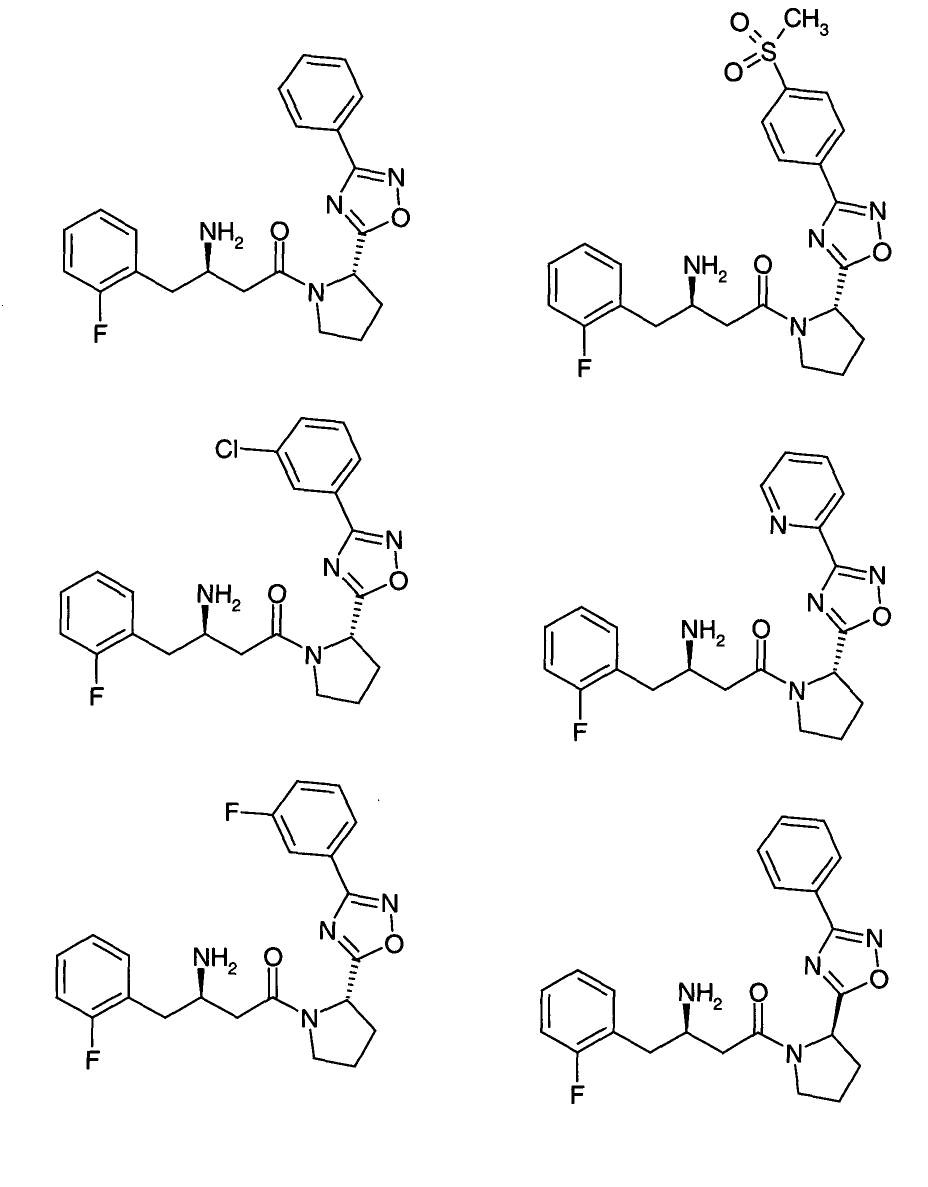

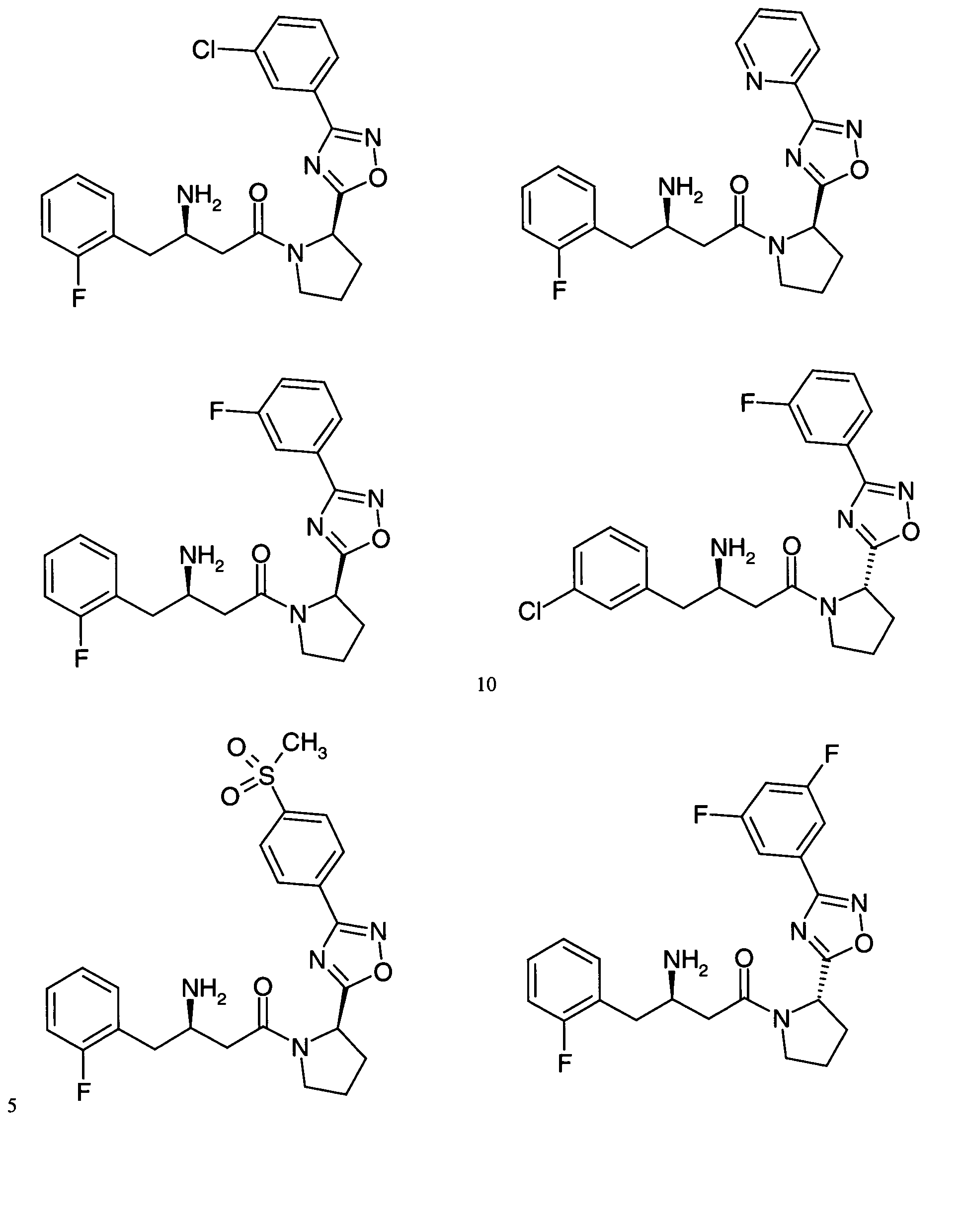

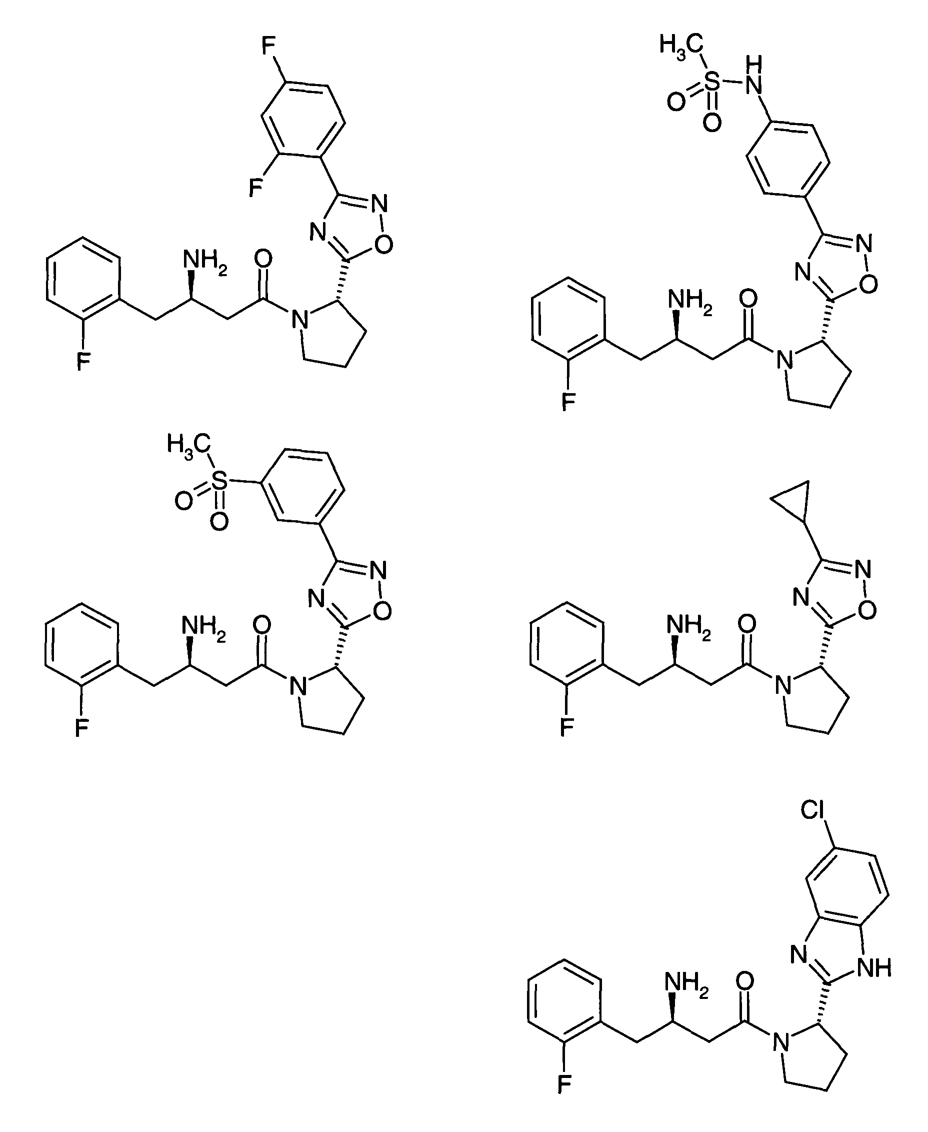

Furthermore, the present invention provides prodrug compounds of the compounds of the invention as described above.
"Prodrug compound" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of the prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods.

Metabolites of compounds of formula (I) or (Ia) are also within the scope of the present invention.

Where tautomerism, like e.g. keto-enol tautomerism, of compounds of general formula (I) or (Ia) or their prodrugs may occur, the individual forms, like e.g. the keto and enol form, are claimed separately and together as mixtures in any ratio. Same applies for stereoisomers, like e.g. enantiomers, cis/trans isomers, conformers and the like. If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I) or (Ia) may be obtained from stereoselective synthesis using optically pure starting materials.

In case the compounds according to formula (I) or (Ia) contain one or more acidic or basic groups, the invention also comprises their corresponding pharmaceutically or toxicologically acceptable salts, in particular their pharmaceutically utilizable salts. Thus, the compounds of the formula (I) or (Ia) which contain acidic groups can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) or (Ia) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) or (Ia) simultaneously contain acidic and basic groups in the molecule, the invention also includes, in addition to the salt forms mentioned, inner salts or betaines (zwitterions). The respective salts according to the formula (I) or (Ia) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I) or (Ia) which, owing to low physiological compatibility, are not directly suitable for use in pharmaceuticals but which can be used, for example, as intermediates for chemical reactions or for the preparation of pharmaceutically acceptable salts.

The present invention provides compounds of general formula (I) or (Ia) or their prodrugs as DPP-IV inhibitors. DPP-IV is a cell surface protein that has been implicated in a wide range of biological functions. It has a broad tissue distribution (intestine, kidney, liver, pancreas, placenta, thymus, spleen, epithelial cells, vascular endothelium, lymphoid and myeloid cells, serum), and distinct tissue and cell-type expression levels. DPP-IV is identical to the T cell activation marker CD26, and it can cleave a number of immunoregulatory, endocrine, and neurological peptides *in vitro.* This has suggested a potential role for this peptidase in a variety of disease processes.

DPP-IV related diseases are described in more detail in WO-A-03/181 under the paragraph "Utilities" which is herewith incorporated by reference.

Accordingly, the present invention provides compounds of formula (I) or (Ia) or their prodrugs or pharmaceutically acceptable salt thereof for use as a medicament.

Furthermore, the present invention provides the use of compounds of formula (I) or (Ia) or their prodrugs or a pharmaceutically acceptable salt thereof for the manufacture of a medicament for the treatment or prophylaxis of non-insulin dependent (Type II) diabetes mellitus; hyperglycemia; obesity; insulin resistance; lipid disorders; dyslipidemia; hyperlipidemia; hypertriglyceridemia; hypercholestrerolemia; low HDL; high LDL; atherosclerosis; growth hormone deficiency; diseases related to the immune response; HIV infection; neutropenia; neuronal disorders; tumor metastasis; benign prostatic hypertrophy; gingivitis; hypertension; osteoporosis; diseases related to sperm motility; low glucose tolerance; insulin resistance; ist sequelae; vascular restenosis; irritable bowel syndrome; inflammatory bowel disease; including Crohn's disease and ulcerative colitis; other inflammatory conditions; pancreatitis; abdominal obesity; neurodegenerative disease; retinopathy; nephropathy; neuropathy; Syndrome X; ovarian hyperandrogenism (polycystic ovarian syndrome; Type n diabetes; or growth hormone deficiency. Preferred is non-insulin dependent (Type II) diabetes mellitus and obesity.

The present invention provides pharmaceutical compositions comprising a compound of formula (I) or (Ia), or a prodrug compound thereof, or a pharmaceutically acceptable salt thereof as active ingredient together with a pharmaceutically acceptable carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

A pharmaceutical composition of the present invention may additionally comprise one or more other compounds as active ingredients like one or more additional compounds of formula (I) or (Ia), or a prodrug compound or other DPP-IV inhibitors.
Other active ingredients are disclosed in WO-A-03/181 under the paragraph "Combination Therapy" which is herewith incorporated by reference.
Accordingly, other active ingredients may be insulin sensitizers; PPAR agonists; biguanides; protein tyrosinephosphatase-IB (PTP-1B) inhibitors; insulin and insulin mimetics; sulfonylureas and other insulin secretagogues; a-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents; HMG-CoA reductase inhibitors; sequestrants; nicotinyl alcohol; nicotinic acid or a salt thereof; PPARa agonists; PPARoly dual agonists; inhibitors of cholesterol absorption; acyl CoA : cholesterol acyltransferase inhibitors; anti-oxidants; PPARo agonists; antiobesity compounds; an ileal bile acid transporter inhibitor; or anti-inflammatory agents or pharmaceutically acceptable salts of these active compounds.

The term "pharmaceutically acceptable salts" refers to salts prepared from pharmaceutically acceptable non-toxic bases or acids, including inorganic bases or acids and organic bases or acids.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of formula (I) or (Ia) can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of formula (I) or (Ia) may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.
Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of formula (I) or (Ia) are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing diabetes mellitus and/or hyperglycemia or hypertriglyceridemia or other diseases for which compounds of Formula I are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligrams to about 50 milligrams. In the case of a 70 kg adult human, the total daily dose will generally be from about 7 milligrams to about 350 milligrams. This dosage regimen may be adjusted to provide the optimal therapeutic response.

Some abbreviations that may appear in this application are as follows.

### ABBREVIATIONS

### Designation

- Boc (or boc): *tert*-Butoxycarbonyl
- CDI: 1,1'-Carbonyldiimidazole
- DCE: Dichloroethane
- DCM: Dichloromethane
- DIC: 1,3-Di-*iso*-propylcarbodiimide
- DIPEA: Di-*iso*-propyl-ethylamine
- DMF: *N,N*-Dimethylformamide
- EDC: 1-Ethyl-3-(3-dimethylaminopropyl)carbodiimide hydrochloride
- Et₃N: Triethylamine
- h: hour(s)
- HATU: O-(7-Azabenzotriazol-1-yl)-*N,N,N*'*N'*-tetramethyluronium hexafluorophosphate
- HOBt: 1-Hydroxybenzotriazole
- HPLC: High pressure liquid chromatography
- min.: minute(s)
- PG (or pg): Protecting group
- rt: Retention time
- TFA: Trifluoroacetic acid

Available starting materials may be suitably *N*-protected amino acids (II), nitriles (III) or aldehydes (IV).

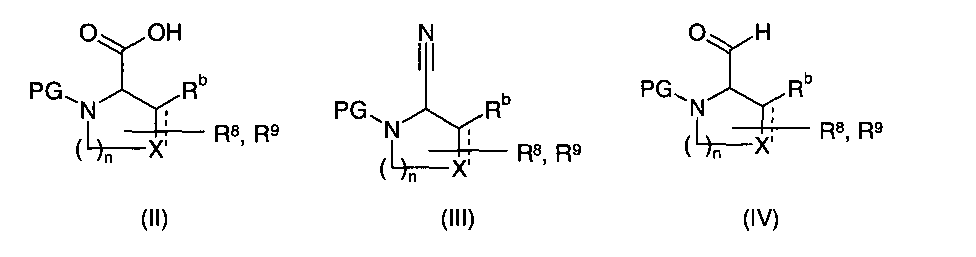

They may be purchased from commercially available sources such as ABCR, Array, Astatech, Sigma-Aldrich, Fluka or be synthesised by
(a) dehydration of the corresponding amide (Scheme A) 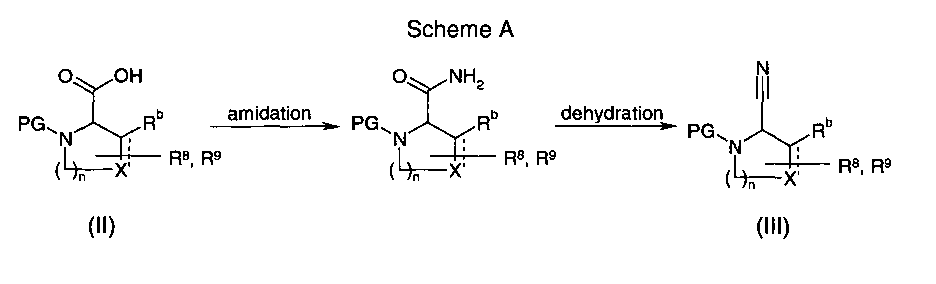
(b) reduction of the Weinreb amide of the carboxylic acid with metal hydride reagents e.g. lithium aluminumhydride (Scheme B).

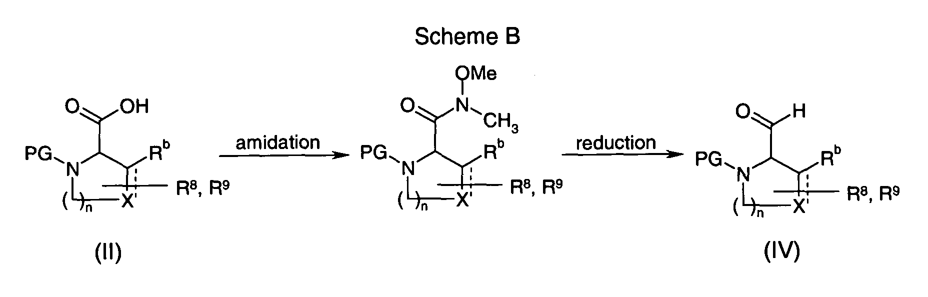

The functional groups of the *N*-protected amino acids may then be transformed into heteroaromatic ring systems by the following reaction sequences:

Reacting an amino acid of the general structure (II) with amidoximes and subsequent dehydration and deprotection can lead to the formation of 1,2,4-oxadiazole compounds of the general type (V), as shown in Scheme C. According to F. Eloy, R. Lenaers, *Chem. Rev.* **1962**, 62, 155, starting amidoximes can be prepared from nitriles by reaction with hydroxylamine. 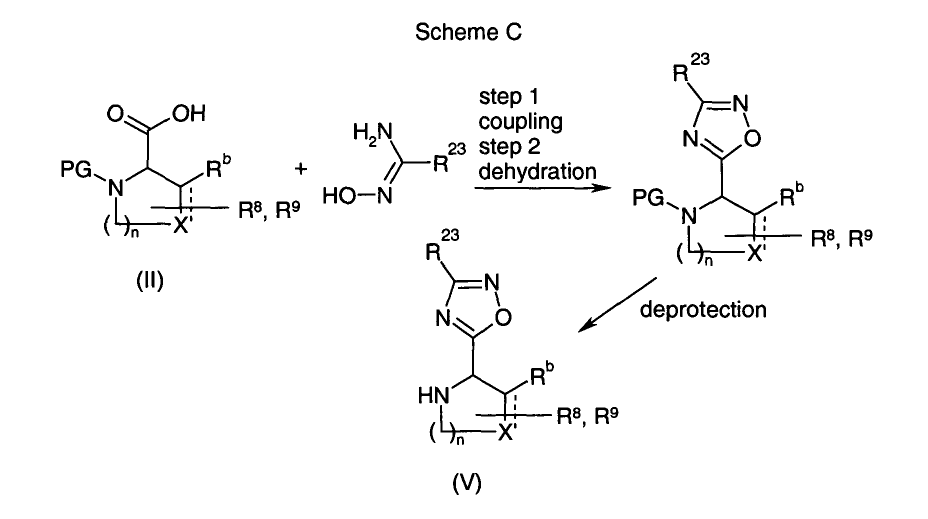 1,2,4-Oxadiazoles with a reversed substitution pattern can be prepared by using a similar reaction scheme starting from nitriles of the general type (III) as described in Scheme D. First, the nitriles are transformed into the amidoximes by reaction with hydroxylamine. The amidoximes are then coupled with carboxylic acids, and the resulting O-acyl amidoximes can be dehydrated to yield the desired 1,2,4-oxadiazoles of the general structure (VI). 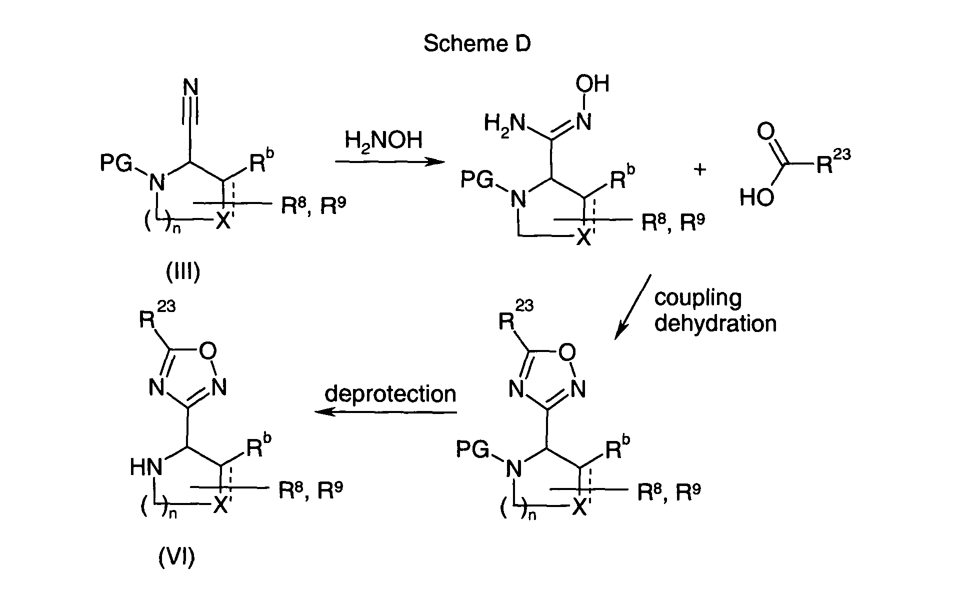 Pyrrazole containing amines of the general structure (VII) may be synthesised according to M. Falorni, G. Giacomelli A. M. Spanedda, *Tetrahedron: Asymmetry* **1998***,* 9, 17, 3039-3046 as shown in Scheme E. Treating Weinreb amides, derived from amino acids of the general type (II), with trimethylsilyethynyl magnesium bromide may lead to the formation of propyn-3-one derivatives which can subsequently be treated with hydrazines to yield pyrazoles.

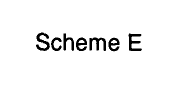

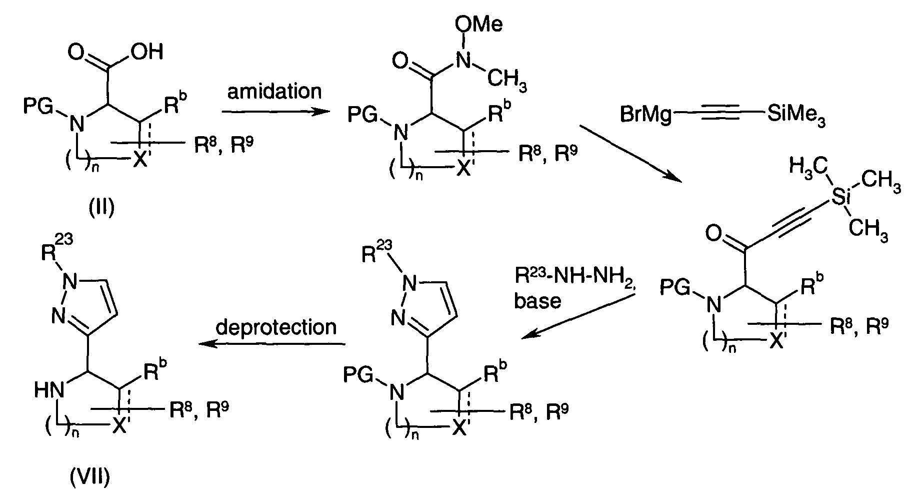

Scheme F depicts a general route towards 1,3,4-oxadiazoles of the structure (VIII) as described e.g. in C. T. Brain, J. M. Paul, Y. Loog, P. J. Oakley, *Tetrahedron Lett.***1999,** 40, 3275-3278. Amino acids of the general type (II) may be transformed into their acid chlorides that upon reaction with acylhydrazines may yield diacylhydrazines. Subsequent dehydration and deprotection can result in the formation of 1,3,4-oxadiazoles of the general type (VIII).

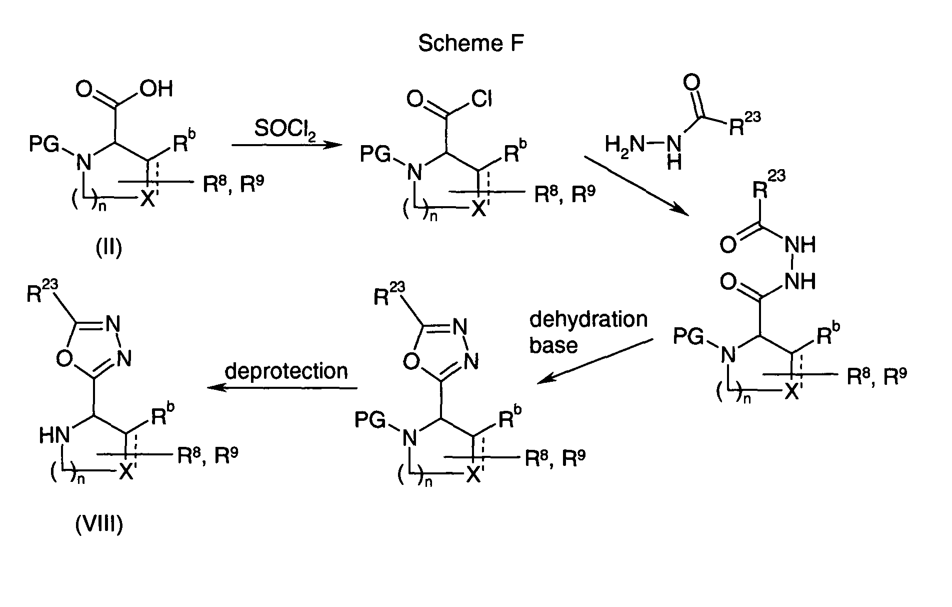

A typical synthesis of 1,3,4-triazoles is shown in Scheme G. Starting from amino acids of the general type (II) 1,3,4-triazoles can be prepared via amides that can be transformed into imidoyl chlorides by e.g. phosphorousoxychloride. Treating these imidoyl chlorides with hydrazides under acidic conditions may lead to the formation of 1,3,4-triazoles that after final deprotection can result in structures of the general type (IX).

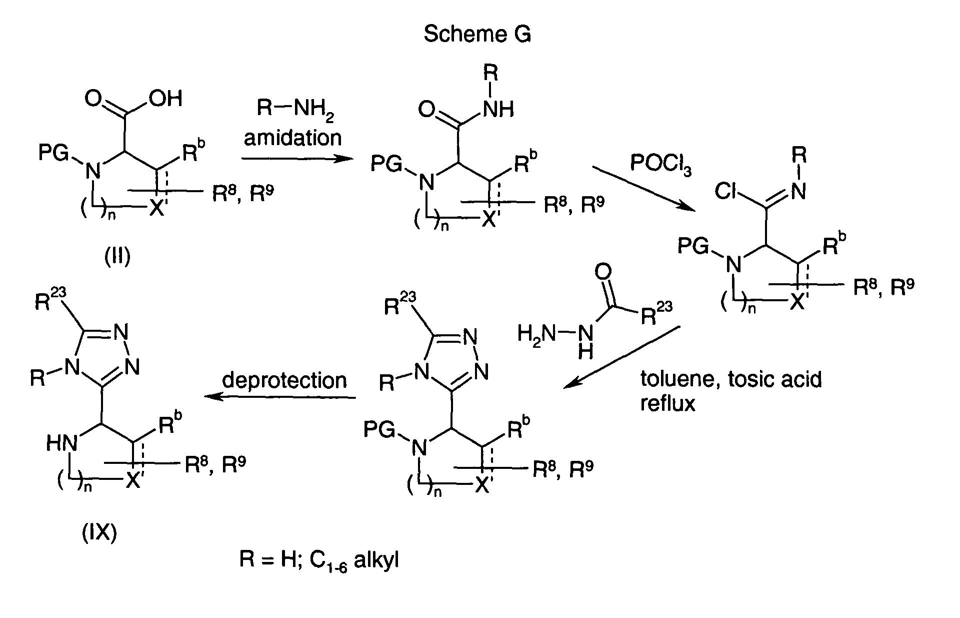

As shown in Scheme H benzimidazoles of the general type (X) may be prepared from aldehydes of the type (IV) by reaction with diamino compounds, followed by oxidation and deprotection.

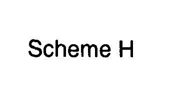

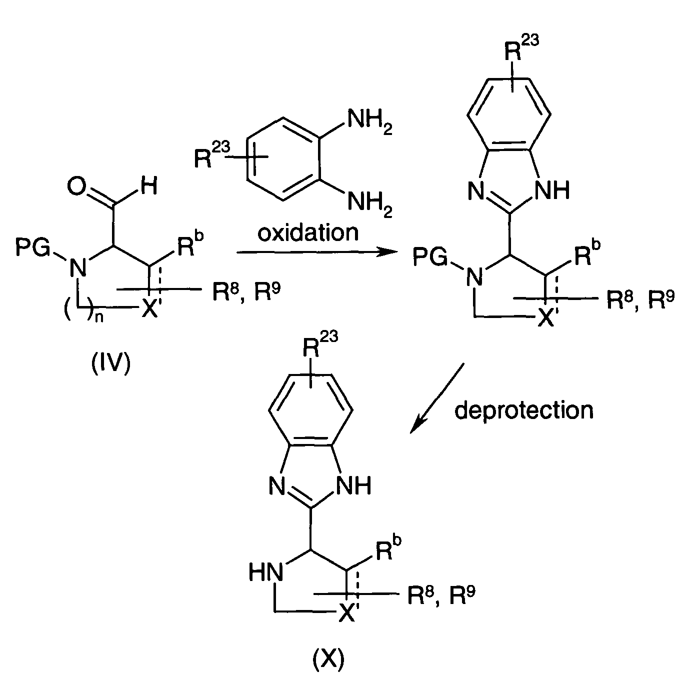

Other heterocyclic compounds may be prepared according to T. Eicher, S. Hauptmann, The Chemistry of Heterocycles, Ed. Wiley-VCH, Weinheim, 2003, or the literature cited therein.

Enantiomerically pure beta amino acids having the formula (XI) 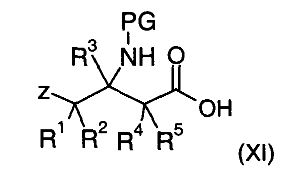 may be commercially available, known in the literature or may be conveniently synthesised using one of the methods already published e.g. reviewed in *Aldrichimica Acta;* 27; 3;1994, or *Enantioselective Synthesis of β-Amino Acids,* Ed. Wiley-VCH, New York; 1997.

Enantiomerically pure beta amino aldehydes having the formula (XII) 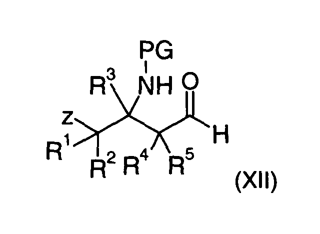 may be commercially available or synthesised by one skilled in the art using as starting material the conveniently substituted acids e.g. through direct reduction with di-*iso*-butylaluminium hydride or through formation of the Weinreb amide and further reduction with lithium aluminiumhydride, as depicted in Scheme I.

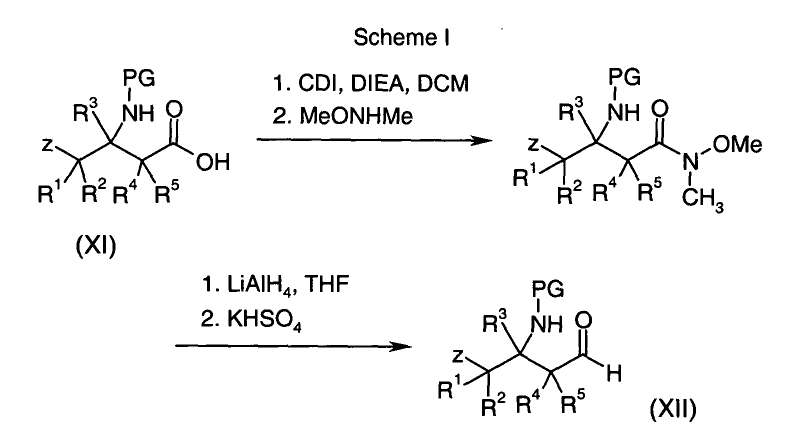

Unless otherwise noted, all non-aqueous reactions were carried out under argon atmosphere with commercial solvents. Compounds were purified using flash column chromatography using Merck silica gel 60 (230-400 mesh) or reverse phase preparative HPLC using a Reprosil-Pur ODS3, 5 µm, 20 x 125 mm column with Shimadzu LC8A-Pump and SPD-10Avp UV/Vis diode array detector. The ¹H-NMR spectra were recorded on a Varian VXR-S (300 MHz for ¹H-NMR) using CDCl₃ or d₆-dimethylsulfoxide as solvent; chemical shifts are reported in ppm relative to tetramethylsilane. Analytical LC/MS was performed using Reprosil-Pur ODS3, 5 µM, 1 x 60 mm columns with a linear gradient from 5% to 95% acetonitrile in water (0.1% TFA) at a flow rate of 250 µl/min; retention times are given in minutes. Methods are: (I) runs on a LC10Advp-Pump (Shimadzu) with SPD-M10Avp UV/Vis diode array detector and QP2010 MS-detector in ESI+ modus with UV-detection at 214, 254 and 275 nm, 5 min. linear gradient; (II) idem but 10 min. linear gradient; (III) runs on a LC10Advp-Pump (Shimadzu) with SPD-10Avp dual wavelength UV-detector and QP2010 MS-detector in ESI+ modus with UV-detection at 214 and 254 nm, 5 min. linear gradient; (IV) idem but 10 min. linear gradient.

### General procedures for making compounds of the invention

In general, compounds having the structure (I) 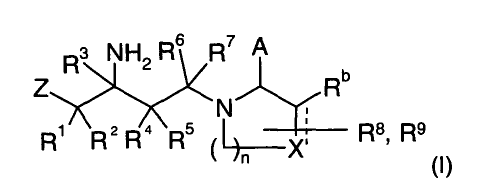 wherein the variables have the above described meanings, may be prepared by
(a) standard peptide coupling reactions. For example, it may be possible to use 1-ethyl-3-(3-dimethylaminopropyl)carbodiimide hydrochloride (EDC) in combination with 1-hydroxybenzotriazole (HOBt) and a base (triethylamine or diisopropylethylamine) or O-(7-azabenzotriazol-1-yl)-*N,N,N',N'*-tetramethyluronium hexafluorophosphate (HATU) in the presence of a base, in solvents such as methylene chloride or *N*,*N*-dimethylformamide. Scheme J outlines a procedure for using the amines formed according to Schemes A through H to synthesize compounds that are embodiments of the invention. 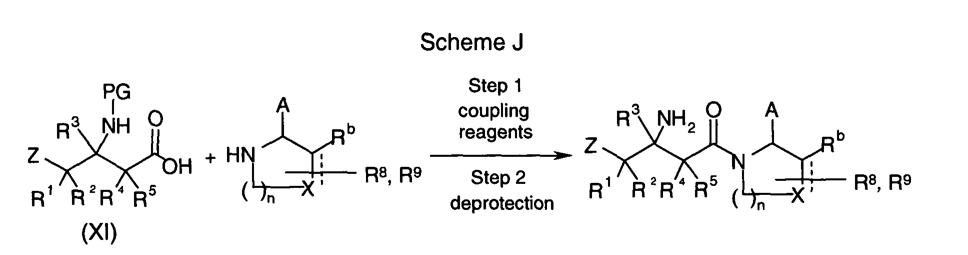 The protective group may be removed with, for example, diethylamine in dichloromethane in the case of 9-fluorenylmethoxycarbonyl or using acidic conditions (such as trifluoroacetic acid in dichloromethane or hydrochloric acid in dioxane) in the case of *tert*-butoxycarbonyl, as described in *Protective Groups in Organic Synthesis* 3^{rd} ed., Ed. Wiley-VCH, New York; 1999.
(b) reduction of suitable amides (eg. with lithium aluminiumhydride in tetrahydrofuran) Scheme K outlines a procedure for using the amides formed according to Scheme J to synthesise compounds that are embodiments of the invention. 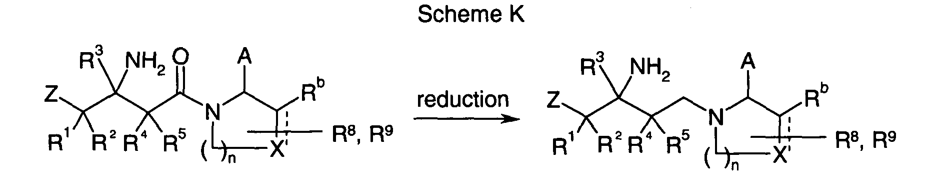
(c) reductive amination reducing the imine formed from a conveniently substituted aldehyde and an amine (eg. in acidic medium with a triacetoxyborohydride salt or sodium borohydride in dichloromethane, methanol or ethanol). Scheme L outlines a procedure for reducing the imines obtained by reacting suitable amines (e.g., prepared according to Schemes A to H) and aldehydes with formula (XII). 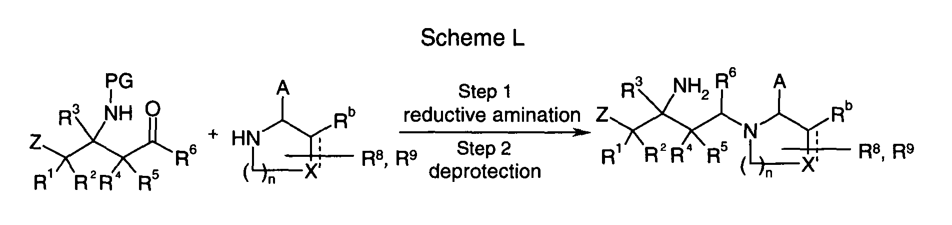
(d) the Kulnikovic reaction with an alkylmagnesium halide in the presence of titanium tetraisopropoxide in aprotic solvents such as tetrahydrofuran. Scheme M outlines a procedure for the use of the Kulnikovic reaction with amides prepared according to Scheme J.

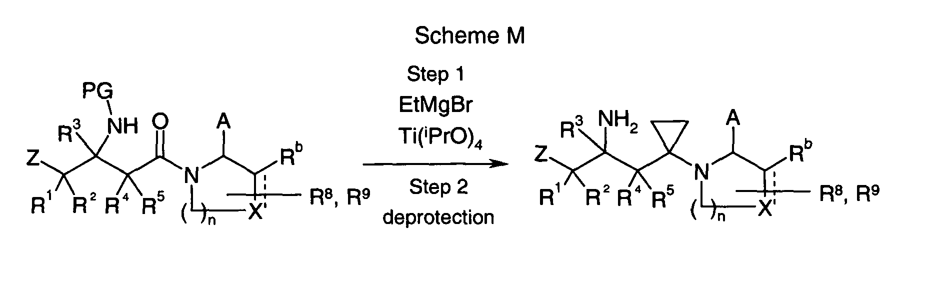

For the purification of intermediates or end products, flash chromatography on silica gel may be suitable for the free amines whereas the use of preparative HPLC leads to the isolation of the corresponding trifluoroacetic acid salts.

Compounds may be prepared by other means however, and the suggested starting materials and procedures described below are exemplary only and should not be considered as limiting the scope of the invention.

### EXAMPLES

The following examples are provided so that the invention might be more fully understood. These examples are illustrative only and should not be construed as limiting the invention in any way.

### PREPARATIONS

### Example 1

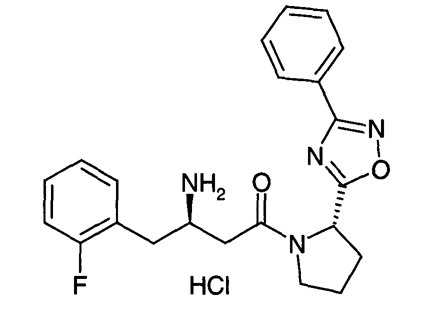

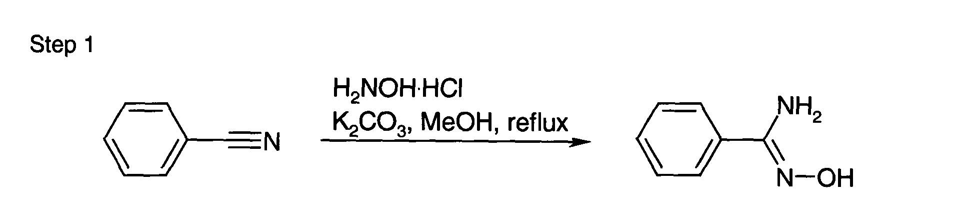

### N-Hydroxy-benzamidine

10.3 g (0.10 mol, 1.00 eq) of benzonitrile are dissolved in 40 mL of methanol, 20.7 g (0.15 mol, 1.50 eq) of powdered potassium carbonate is added and 13.9 g (0.20 mol, 2.00 eq) of hydroxylamine hydrochloride dissolved in 120 mL of methanol is added subsequently in small portions. The reaction mixture is refluxed for 5 h then the solvent is evaporated and the residue is taken up in a 1:4 mixture of water and chloroform. The organic layer is separated, washed twice with water, dried with magnesium sulfate, filtered and evaporated to dryness. The residue is recrystallized from ether/hexane to afford the desired product.

Mp.: 77 °C - 79 °C.

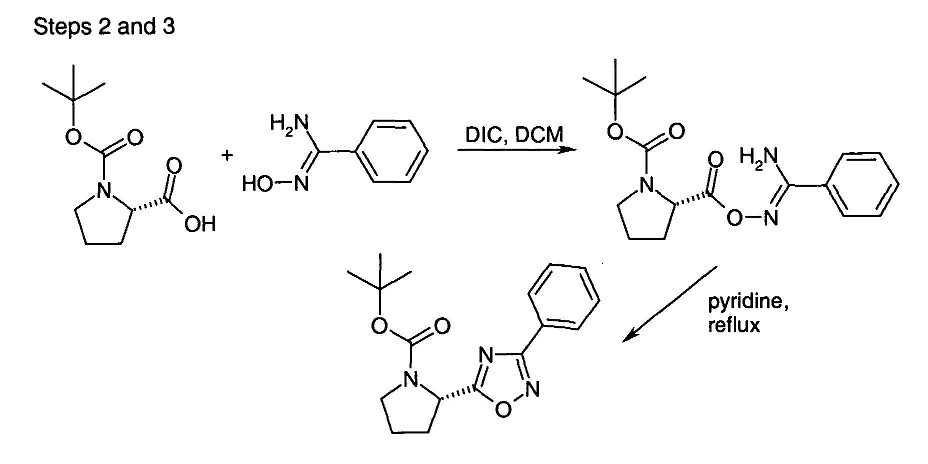

### 2-(3-Phenyl-[1,2,4]oxadiazol-5-yl)-(S)-pyrrolidine-1-carboxylic acid tert-butyl ester

To a solution of 5.75 g (42.2 mmol, 1.00 eq) *N*-hydroxy-benzamidine, 10.0 g (46.5 mmole, 1.10 eq) Boc-(*S*)-proline in 60 mL of dichloromethane, 6.44 g (51.0 mmol, 1.20 eq) of 1,3-di-*iso*-propylcarbodiimide is added and the mixture is stirred at room temperature. After 12 h the mixture is evaporated to dryness and the residue is dissolved in 60 mL of pyridine and refluxed for 10 h. Then the pyridine is evaporated and the residue is taken up in a 2:1 mixture of dichloromethane and water. The aqueous phase is extracted with dichloromethane, and the combined organic layers are washed with 3 % hydrochloric acid solution, saturated sodium bicarbonate solution, water and brine, dried with magnesium sulfate, filtered and concentrated in vacuo. The residue is subjected to column chromatography (silica gel, eluent: *c*-hexane:ethyl acetate = 60:40) to afford the title compound.

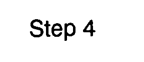

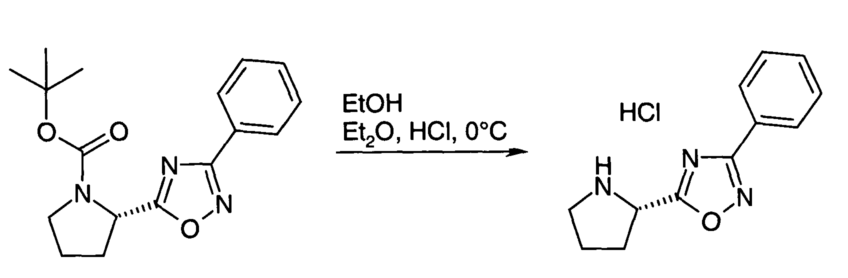

### 3-Phenyl-5-(S)-pyrrolidin-2-yl-[1,2,4]oxadiazole hydrochloride

The Boc-protected intermediate from step 3 is dissolved in 40 mL of ethanol and cooled to 0°C. Then 40 mL of diethylether saturated with HCl gas is added and the reaction mixture is allowed to warm to room temperature. After complete conversion of the starting material, as monitored by TLC (silica gel, eluent: dichloroethane-ethanol 4:1), the solvents are evaporated. The residue is taken up in diethylether and the precipitate is filtered off, washed with diethylether and dried in vacuo to yield the title compound.
¹H-NMR (300 MHz, CDCl₃) δ = 11.18 (s, 1 H, NH), 10.24 (s, 1 H, NH), 8.04 (m, 2H, aryl-H), 7.55-7.4 (m, 3H, aryl-H), 5.2 (m, 1H, CH), 3.64 (m, 2H, CH₂), 2.65-2.15 (m, 4H, 2xCH₂).

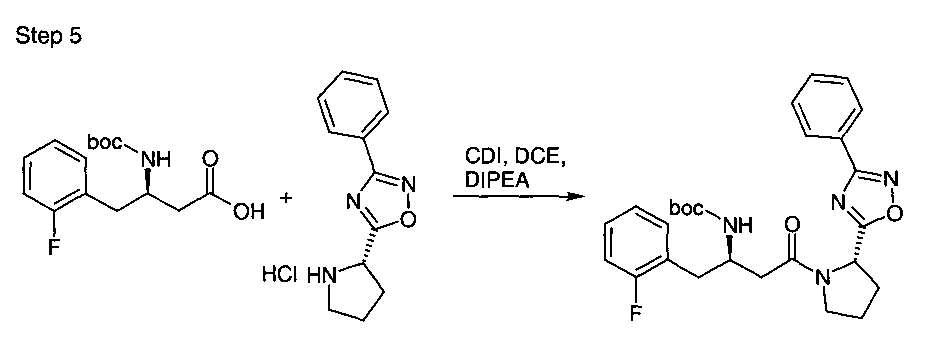

### (R)-{1-(2-Fluoro-benzyl)-3-oxo-3-[2-(3-phenyl-[1,2,4]oxadiazol-5-yl)-(S)-pyrrolidin-1-yl]-propyl}-carbamic acid tert-butyl ester

A mixture of 125 mg (0.42 mmol, 1.00 eq) of Boc-(*R*)-3-amino-4-(2-fluoro-phenyl)-butyric acid and 71.4 mg (0.44 mmol, 1.05 eq) of 1,1'-carbonyldiimidazole in 5.00 mL of 1,2-dichloroethane was stirred for 2 h. Separately, 106 mg (0.42 mmol, 1.05 eq) of 3-Phenyl-5-(*S*)-pyrrolidin-2-yl-[1,2,4]oxadiazole hydrochloride (step 4) and (0.46 mmole, 1.10 eq) of *N*,*N*-di-*iso*-propylethylamine are stirred in 4.00 mL of 1,2-dichloroethane. After 15 min. the solution of the amine is added to the mixture of the activated acid via a syringe and stirring is continued overnight at room temperature. Then the reaction mixture is heated to reflux for two days. The solvent is evaporated and the residue is taken up in a 1:1 mixture of dichloromethane and water. The aqueous layer is extracted with dichloromethane and the combined organic layers are washed sequentially with 5 % citric acid solution, 10 % sodium carbonate solution, water and brine, dried with magnesium sulfate, filtered and concentrated in vacuo. Purification by preparative thin layer-chromatography on silica gel affords the title compound.

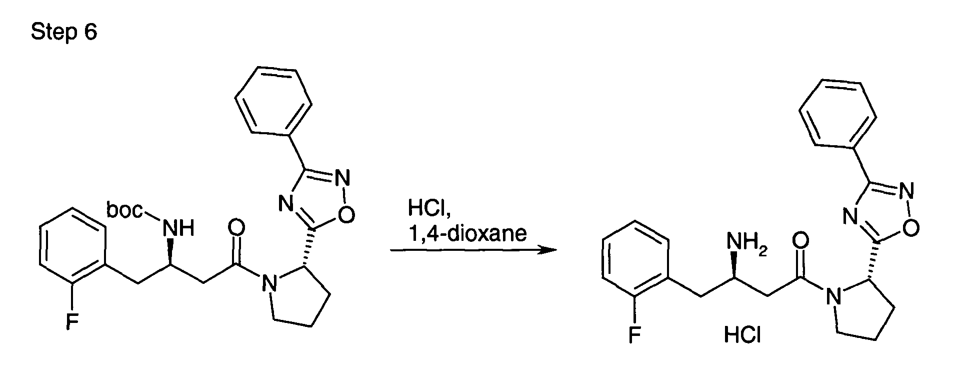

### (R)-3-Amino-4-(2-fluoro-phenyl)-1 -[(S)-2-(3-phenyl-[1,2,4]oxadiazol-5-yl)-pyrrolidin-1-yl]-butan-1-one hydrochloride

The Boc-protected intermediate from step 5 is dissolved at 0°C in 15 mL of 1,4-dioxane that has previously been saturated with HCl gas. The reaction mixture is allowed to warm to room temperature over the course of the reaction and stirring is continued until complete conversion is observed by TLC analysis. Evaporation of the solvents affords a solid residue, which is taken up in a mixture of ether and hexane. The precipitated final compound is filtered off, washed with hexane and dried in vacuo.

¹H-NMR (300 MHz, CDCl₃) δ = 8.65 (m, 3H, NH), 8.0 (m, 2H, aryl-H), 7.45 (m, 3H, aryl-H), 7.4 (m, 1 H, aryl-H), 7.24 (m, 1H, aryl-H), 7.1 (m, 2H, aryl-H), 5.42 (m, 1 H, CH), 4.00-3.40 (m, 3H, CH and CH₂), 3.1-2.0 (m, 8H, 4xCH₂).

Using this general procedure the following oxadiazole derivatives were prepared:

### Example 2

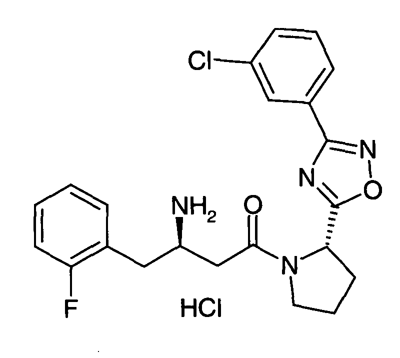

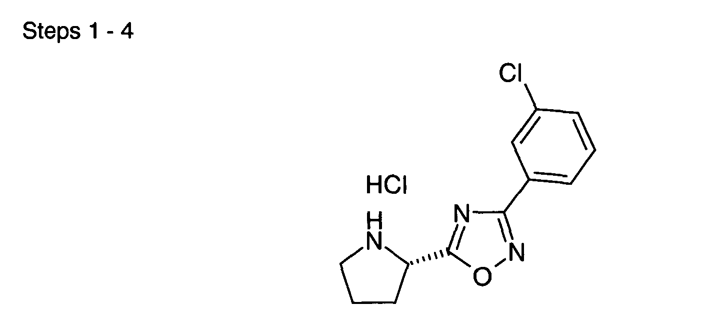

### 3-(3-Chloro-phenyl)-5-(S)-pyrrolidin-2-yl-[1,2,4]oxadiazole hydrochloride

The title compound was prepared according to steps 1 - 4 of example 1.

¹H-NMR (300 MHz, CDCl₃) δ = 11.2-10.2 (s, 2H, NH), 8.04 (dd, 1H, aryl-H), 7.94 (ddd, 1 H, aryl-H), 7.51 (ddd, 1 H, aryl-H), 7.41 (dd, 1 H, aryl-H), 5.2 (m, 1 H, CH), 3.8-3.6 (m, 2H, CH), 2.7 (m, 1H, CH), 2.4 (m, 1H, CH), 2.21 (m, 2H, CH₂).

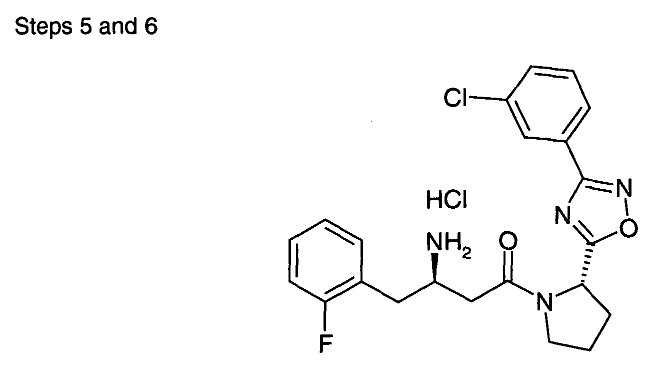

### (R)-3-Amino-1-{(S)-2-[3-(3-chloro-phenyl) [1,2,4]oxadiazol-5-yl)-pyrrolidin-1-yl}-4-(2-fluoro-phenyl)-butan-1-one hydrochloride

The title compound was prepared according to steps 5 and 6 of example 1.

¹H-NMR (300 MHz, CDCl₃) δ = 8.7 (m, 3H, NH₃), 8.0 (dd, 1H, aryl-H), 7.9 (m, 1H, aryl-H), 7.5-7.0 (m, 6H, aryl-H), 5.1 (m, 1H, CH), 3.92 (m, 1H, CH), 3.5 (m, 2H, CH₂), 3.21-2.2 (m, 4H, 2xCH₂).

### Example 3

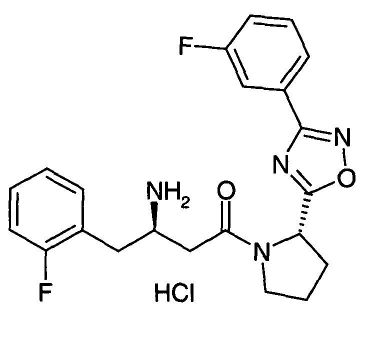

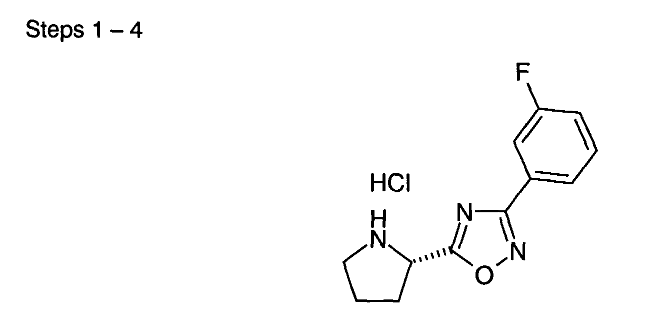

### 3-(3-Fluoro-phenyl)-5-(S)-pyrrolidin-2-yl-[1,2,4]oxadiazole hydrochloride

The title compound was prepared according to steps 1 - 4 of example 1.

¹H-NMR (300 MHz, CDCl₃) δ = 11.2-10.4 (s, 2H, NH₂), 7.86 (m, 1H, aryl-H), 7.76 (m, 1 H, aryl-H), 7.43 (m, 1H, aryl-H), 7.20 (m, 1 H, aryl-H), 5.2 (m, 1 H, CH), 3.75-3.64 (m, 2H, CH₂), 2.60-2.45 (m, 2H, CH₂), 2.32-2.17 (m, 2H, CH₂).

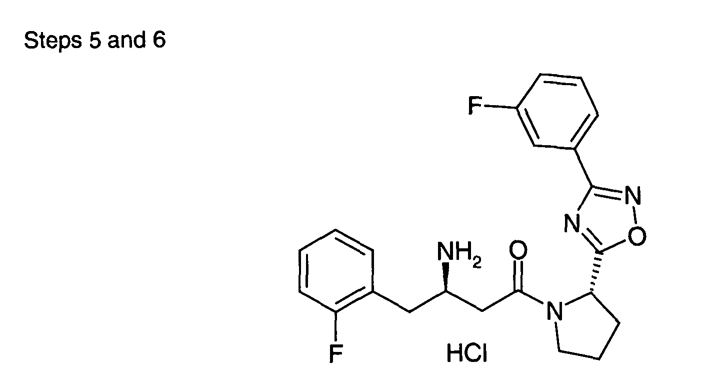

### (R)-3-Amino-1-{(S)-2-[3-(3-fluoro-phenyl) [1,2,4]oxadiazol-5-yl)-pyrrolidin-1-yl}-4-(2-fluoro-phenyl)-butan-1-one hydrochloride

The title compound was prepared according to steps 5 and 6 of example 1.

¹H-NMR (300 MHz, CDCl₃) δ = 8.15 (m, 3H, NH₃), 7.85 (m, 1 H, aryl-H), 7.70 (m, 1 H, aryl-H), 7.6 (m, 1H, aryl-H), 7.46 (m, 1H, aryl-H), 7.39-7.10 (m, 4H, aryl-H), 5.3 (m, 1H, CH), 3.75 (m, 2H, CH₂), 3.68 (m, 1H, CH), 3.58-2.0 (m, 8H, 4xCH₂).

### Example 4

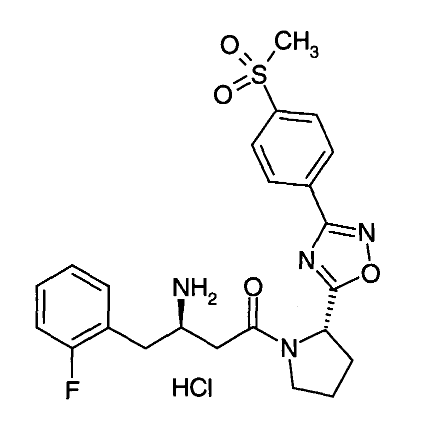

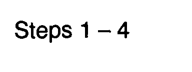

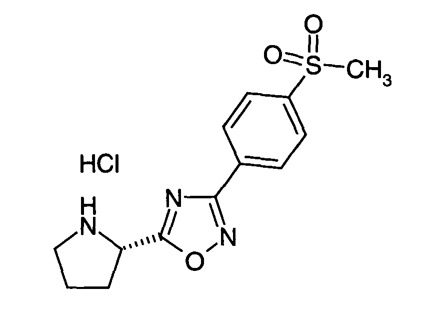

### 3-(4-Methanesulfonyl-phenyl)-5-(S)-pyrrolidin-2-yl-[1,2,4]oxadiazole hydrochloride

The title compound was prepared according to steps 1 - 4 of example 1.

¹H-NMR (300 MHz, CDCl₃ + DMSO-d₆) δ = 10.3 (s, 2H, NH), 8.3-8.15 (m, 4H aryl-H), 5.2 (m, 1H, CH), 3.46 (m, 2H, CH₂), 3.23 (s, 3H, CH₃), 2.65-2.10 (m, 4H, 2xCH₂).

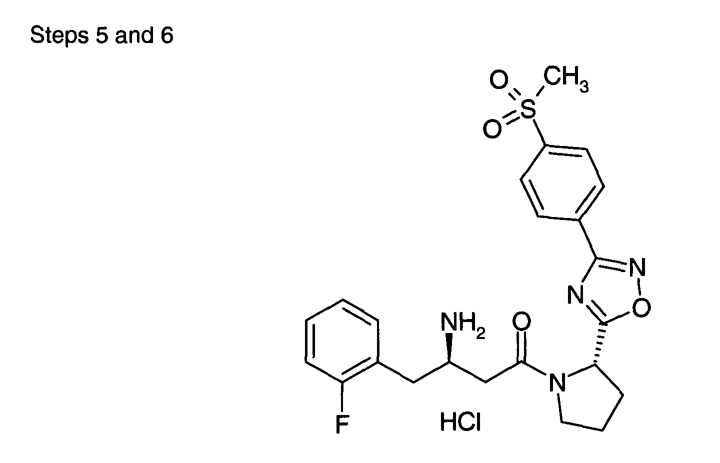

### (R)-3-Amino-4-(2-fluoro-phenyl)-1-{(S)-2-[3-(4-methanesulfonyl-phenyl) [1,2,4]oxadiazol-5-yl]-pyrrolidin-1-yl}-butan-1-one hydrochloride

The title compound was prepared according to steps 5 and 6 of example 1.

¹H-NMR (300 MHz, DMSO-d₆) δ = 8.4 (m, 3H, NH₃), 8.25-8.08 (m, 4H, aryl-H), 7.4-7.1 (m, 4H, aryl-H), 5.3 (m, 1H, CH), 3.7 (m, 2H, CH₂), 3.6 (m, 1H, CH), 3.28 (s, 3H, CH₃), 3.15-2.6 (m, 4H, 2xCH₂), 2.35-1.9 (m, 4H, 2xCH₂).

### Example 5

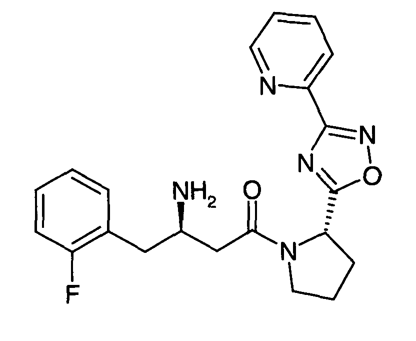

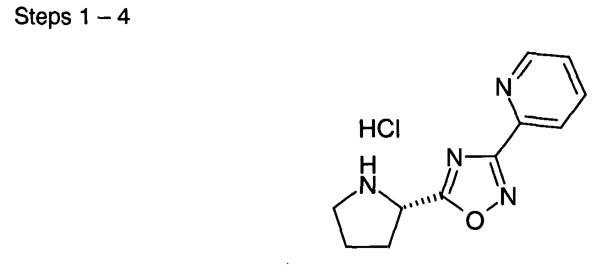

### (S)-2-(5-Pyrrolidin-2-yl-[1,2,4]oxadiazol-3-yl)-pyridine hydrochloride

The title compound was prepared according to steps 1 - 4 of example 1.

¹H-NMR (300 MHz, CDCl₃ + DMSO-d₆) δ = 11.0-9.8 (s, 2H, NH₂), 8.78 (dd, 1 H, py-H), 8.16 (ddd, 1H, py-H), 8.02 (ddd, 1H, py-H), 7.6 (ddd, 1H, py-H), 5.22 (m, 1H, CH), 3.48 (m, 2H, CH₂), 2.62 (m, 1H, CH), 2.44 (m, 1H, CH), 2.21 (m, 2H, CH₂).

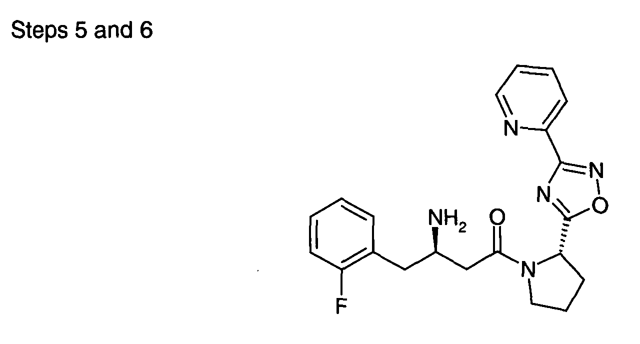

### (R)-3-Amino-4-(2-fluoro-phenyl)-1-[(S)-2-(3-pyridin-2-yl-[1,2,4]oxadiazol-5-yl)-pyrrolidin--yl]-butan-1-one

The title compound was prepared according to steps 5 and 6 of example 1.

¹H-NMR (300 MHz, CDCl₃) δ = 8.78 (dd, 1 H, py-H), 8.12 (dd, 1 H, py-H), 7.82 (m, 1 H, py-H), 7.42 (m, 1 H, py-H), 7.28-7.03 (m, 4H, aryl-H), 5.21 (m, 1 H, CH), 3.77 (m, 1 H, CH), 3.48 (m, 2H, CH₂), 2.83-2.77 (m, 4H, 2xCH₂), 2.57-2.38 (m, 4H, 2xCH₂), 1.68 (m, 2H, NH₂).

### Example 6

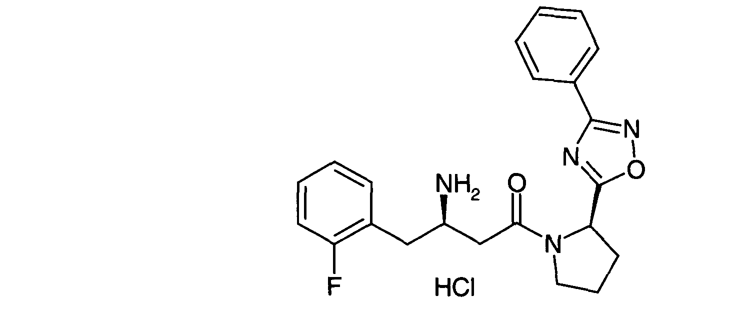

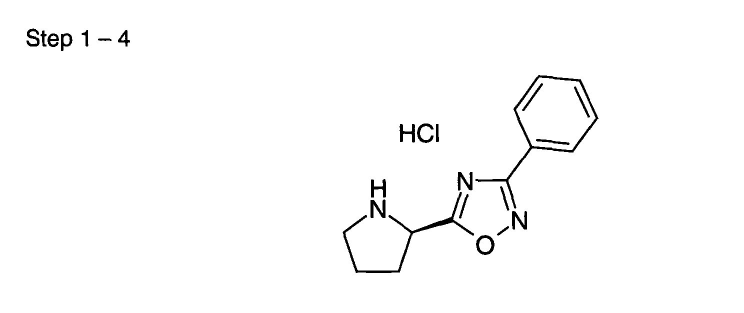

### 3-Phenyl-5-(R)-pyrrolidin-2-yl-[1,2,4]oxadiazole hydrochloride

Steps 1 - 4 were performed according to the procedures described in example 1 with the exception that Boc-(*R*)-proline was used instead of Boc-(*S*)-proline.

¹H-NMR (300 MHz, DMSO-d₆) δ = 11.22 (s, 1 H, NH), 10.25 (s, 1 H, NH), 8.06 (m, 2H, aryl-H), 7.55-7.4 (m, 3H, aryl-H), 5.2 (m, 1H, CH), 3.64 (m, 2H, CH₂), 2.65-2.15 (m, 4H, 2xCH₂).

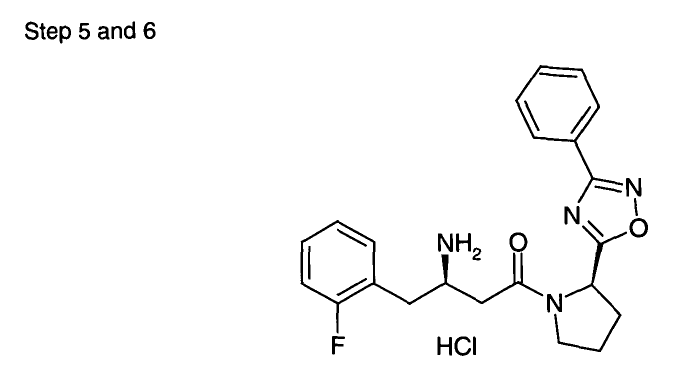

### (R)-3-Amino-4-(2-fluoro-phenyl)-1-[(R)]-2-(3-phenyl-[1,2,4]oxadiazol-5-yl)-pyrrolidin-1-yl]-butan-1-one hydrochloride

The title compound was prepared according to steps 5 and 6 of example 1.

¹H-NMR (300 MHz, DMSO-d₆) δ = 8.3 (m, 3H, NH₃), 8.02-7.90 (m, 2H, aryl-H), 7.65-7.50 (m, 3H, aryl-H), 7.4-6.9 (m, 4H, aryl-H), 5.18 (m, 1 H, CH), 3.73 (m, 1 H, CH), 3.5 (m, 2H, CH₂), 3.18-2.0 (m, 8H, 4xCH₂).

### Example 7

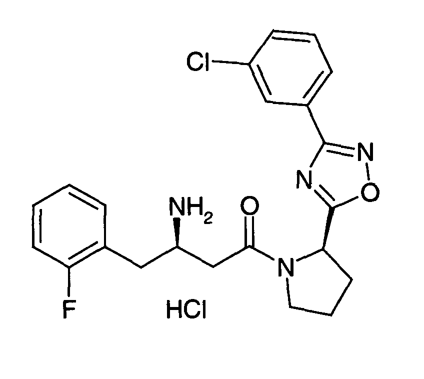

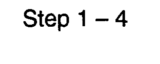

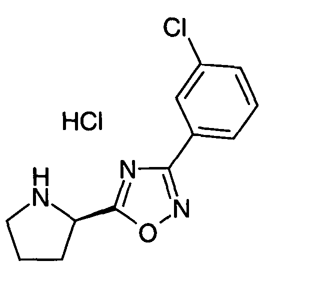

### 3-(3-Chloro-phenyl)-5-(R)-pyrrolidin-2-yl-[1,2,4]oxadiazole hydrochloride

Steps 1 - 4 were performed according to the procedures described in example 1 with the exception that Boc-(*R*)-proline was used instead of Boc-(*S*)-proline.

¹H-NMR (300 MHz, CDCl₃) δ = 10.3 (s, 2H, NH₂), 8.07 (dd, 1H, aryl-H), 8.02 (ddd, 1 H, aryl-H), 7.65 (ddd, 1H, aryl-H), 7.6 (dd, 1H, aryl-H), 5.2 (m, 1H, CH), 3.45 (m, 2H, CH₂), 2.58 (m, 1H, CH), 2.4 (m, 1H, CH), 2.18 (m, 2H, CH₂).

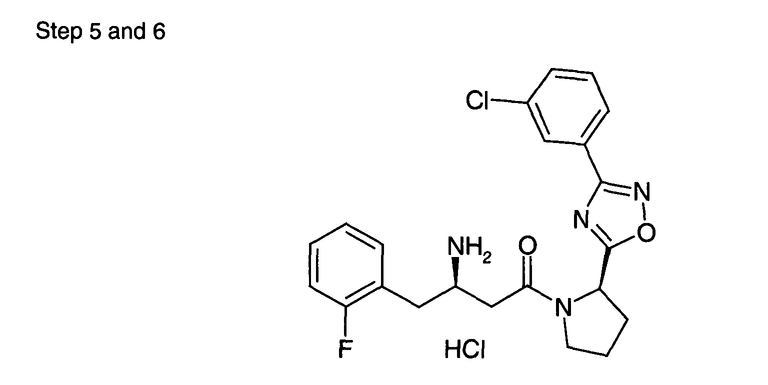

### (R)-3-Amino-1-{(R)-2-[3-(3-chloro-phenyl) [1,2,4]oxadiazol-5-yl)-pyrrolidin-1-yl}-4-(2-fluoro-phenyl)-butan-1-one hydrochloride

The title compound was prepared according to steps 5 and 6 of example 1.

¹H-NMR (300 MHz, CDCl₃) δ = 8.8 (m, 3H, NH₃), 8.1 (dd, 1 H, aryl-H), 7.9 (m, 1 H, aryl-H), 7.5-6.8 (m, 6H, aryl-H), 5.2 (m, 1 H, CH), 3.95 (m, 1H, CH), 3.5 (m, 2H, CH₂), 3.21-2.2 (m, 8H, 4xCH₂).

### Example 8

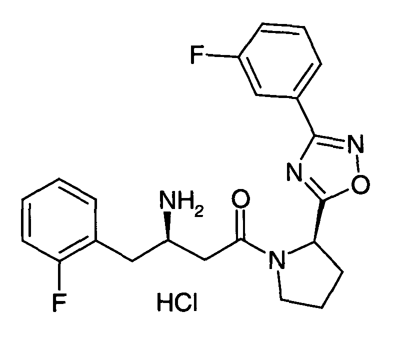

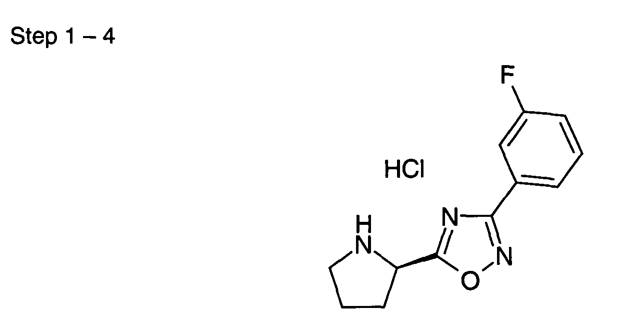

### 3-(3-Fluoro-phenyl)-5-(R)-pyrrolidin-2-yl-[1,2,4]oxadiazole hydrochloride

Steps 1- 4 were performed according to the procedures described in example 1 with the exception that Boc-(*R*)-proline was used instead of Boc-(*S*)-proline.

¹H-NMR (300 MHz, CDCl₃) δ = 11.2 (s, 1H, NH), 10.35 (s, 1H, NH), 7.86 (m, 1H, aryl-H), 7.75 (m, 1 H, aryl-H), 7.43 (m, 1 H, aryl-H), 7.21 (m, 1 H, aryl-H), 5.2 (m, 1 H, CH), 3.65 (m, 2H, CH₂), 2.62-2.45 (m, 2H, CH₂), 2.23 (m, 2H, CH₂).

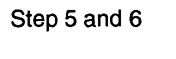

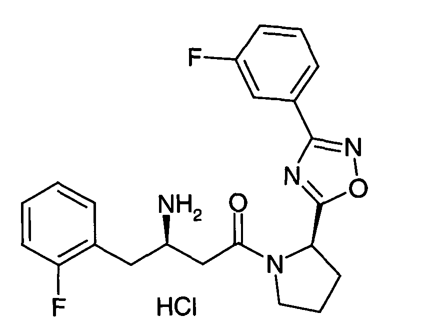

### (R)-3-Amino-1-{(R)-2-[3-(3-fluoro-phenyl) [1,2,4]oxadiazol-5-yl)-pyrrolidin-1-yl}-4-(2-fluoro-phenyl)-butan-1-one hydrochloride

The title compound was prepared according to steps 5 and 6 of example 1.

¹H-NMR (300 MHz, DMSO-d₆) δ = 8.2 (m, 3H, NH₃), 7.78 (m, 1H, aryl-H), 7.67 (m, 1H, aryl-H), 7.63 (m, 1 H, aryl-H), 7.46 (m, 1H, aryl-H), 7.41-7.33 (m, 4H, aryl-H), 5.17 (m, 1H, CH), 3.77 (m, 1H, CH), 3.6 (m, 2H, CH₂), 3.58-2.0 (m, 8H, 4xCH₂).

### Example 9

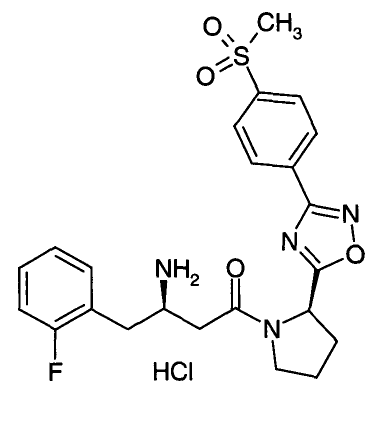

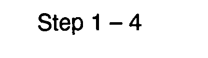

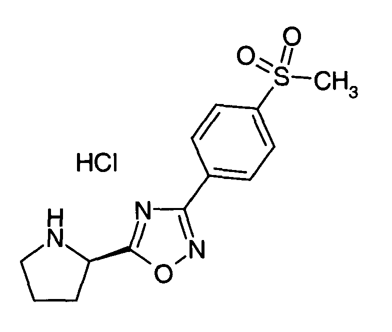

### 3-(4-Methanesulfonyl-phenyl)-5-(R)-pyrrolidin-2-yl-[1,2,4]oxadiazole hydrochloride

Steps 1 - 4 were performed according to the procedures described in example 1 with the exception that Boc-(*R*)-proline was used instead of Boc-(*S*)-proline.

¹H-NMR (300 MHz, CDCl₃ + DMSO-d₆) δ = 11.0-10.0 (s, 2H, NH₂), 8.3-8.15 (m, 4H, aryl-H), 5.2 (m, 1 H, CH), 3.46 (m, 2H, CH₂), 3.21 (s, 3H, CH₃), 2.65-2.10 (m,4H, 2xCH₂).

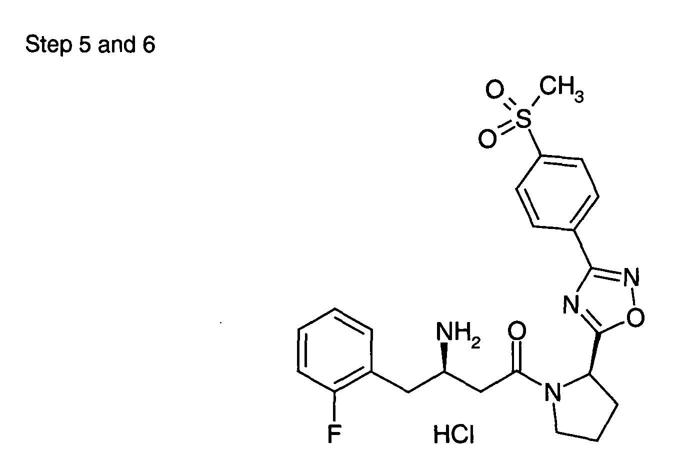

### (R)-3-Amino-4-(2-fluoro-phenyl)-1-{(R)-2-[3-(4-methanesulfonyl-phenyl) [1,2,4]oxadiazol-5-yl]-pyrrolidin-1-yl}-butan-1-one hydrochloride

The title compound was prepared according to steps 5 and 6 of example 1.

¹H-NMR (300 MHz, DMSO-d₆) δ = 8.4 (m, 3H, NH₃), 8.25-8.08 (m, 4H, aryl-H), 7.4-7.1 (m, 4H, aryl-H), 5.2 (m, 1 H, CH), 3.7 (m, 2H, CH₂), 3.6 (m, 1 H, CH), 3.3 (s, 1 H, CH₃), 3.1-2.6 (m, 4H, 2xCH₂), 2.35-1.9 (m, 4H, 2xCH₂).

### Example 10

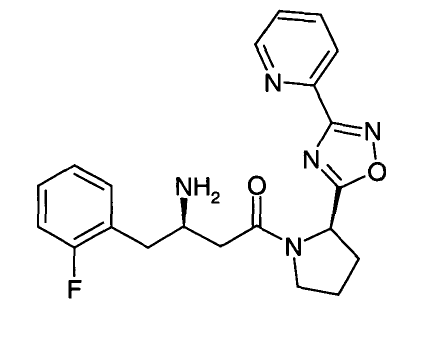

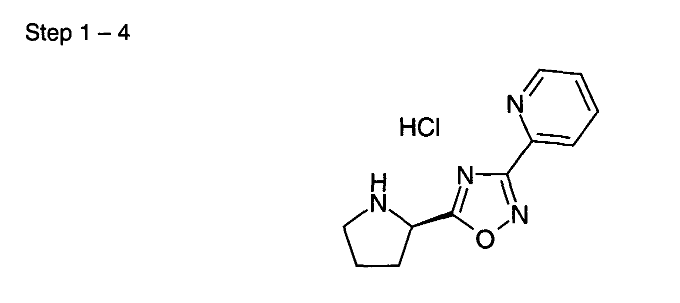

### 2-(5-(R)-Pyrrolidin-2-yl-[1,2,4]oxadiazol-3-yl)-pyridine hydrochloride

Steps 1 - 4 were performed according to the procedures described in example 1 with the exception that Boc-(*R*)-proline was used instead of Boc-(*S*)-proline.

¹H-NMR (300 MHz, CDCl₃ + DMSO-d₆) δ = 10.5 (s, 2H, NH₂), 8.78 (dd, 1H, py-H), 8.16 (ddd, 1 H, py-H), 8.02 (ddd, 1 H, py-H), 7.6 (ddd, 1 H, py-H), 5.22 (m, 1 H, CH), 3.44 (m, 2H, CH₂), 2.6 (m, 1H, CH), 2.42 (m, 1H, CH), 2.21 (m, 2H, CH₂).

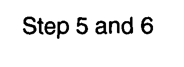

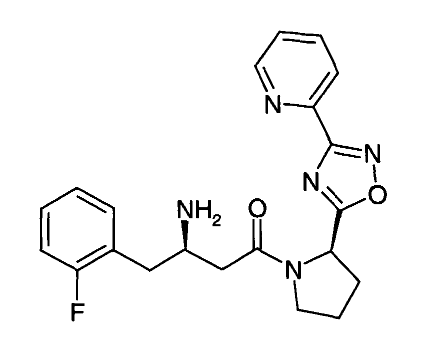

### (R)-3-Amino-4-(2-fluoro-phenyl)-1-[(R)-2-(3-pyridin-2-yl-[1,2,4]oxadiazol-5-yl)-pyrrolidin-1-yl]-butan-1-one

The title compound was prepared according to steps 5 and 6 of example 1.

¹H-NMR (300 MHz, DMSO-d₆) δ = 8.8 (dd, 1 H, py-H), 8.2-7.82 (m, 2H, py-H), 7.6 (m, 1 H, py-H), 7.4-6.9 (m, 4H, aryl-H), 5.22 (m, 1H, CH), 3.8 (m, 1 H, CH), 3.6 (m, 2H, CH₂), 2.8-1.9 (m, 8H, 4xCH₂), 2.0 (m, 2H, NH₂)-

### Example 11

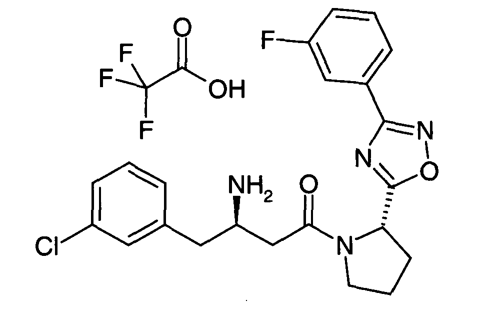

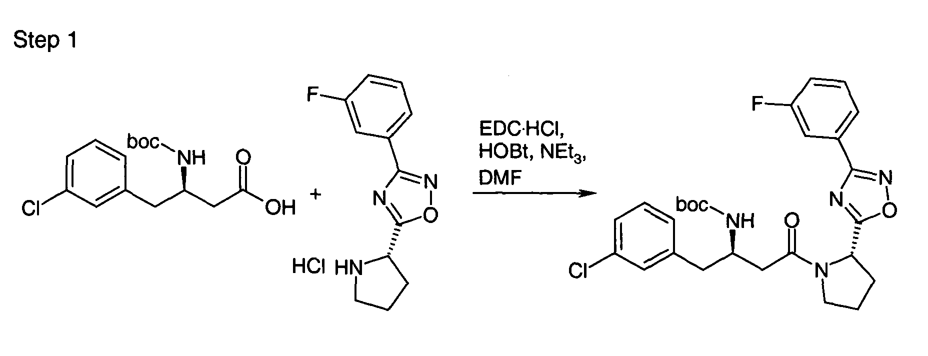

### (R)-(1-(3-Chloro-benzyl-3-{(S)-2-[3-fluoro-phenyl)-[1,2,4]oxadiazol-5-yl]-pyrrolidin-1-yl}-3-oxo-propyl)-carbamic acid tert-butyl ester

25.0 mg (79.7 mmol, 1.00 eq) of Boc-(*R*)-3-amino-4-(3-chloro-phenyl)-butyric acid and 21.5 mg (79.7 mmol, 1.00 eq) of 3-(3-Fluoro-phenyl)-5-(*S*)-pyrrolidin-2-yl-[1,2,4]oxadiazole hydrochloride (prepared according to example 3, steps 1-4) are dissolved in 2.00 mL of DMF. Then 16.1 mg (120 mmol, 1.50 eq) 1-hydroxybenzotriazole, 55.0 µL (39.9 mg, 394 mmol, 3.30 eq) of triethylamine and 22.9 mg (120 mmol, 1.50 eq) of 1-ethyl-3-(3-dimethylaminopropyl)carbodiimide hydrochloride are added to the reaction mixture at room temperature. After the resulting solution is stirred for 12 h at room temperature the mixture is poured into brine and is diluted with water. The aqueous mixture is extracted 3 times with ethyl acetate and the combined organic layers are dried with sodium sulphate. The solvent is removed in vacuo and the title compound is purified by flash chromatography (silica gel, eluent: *c*-hexane:ethyl acetate = 1:1).

LCMS (I) rt 3.60, m/z 529 (M+H)⁺, 470 (M-*t*Bu)⁺, 429 (M-Boc)⁺.

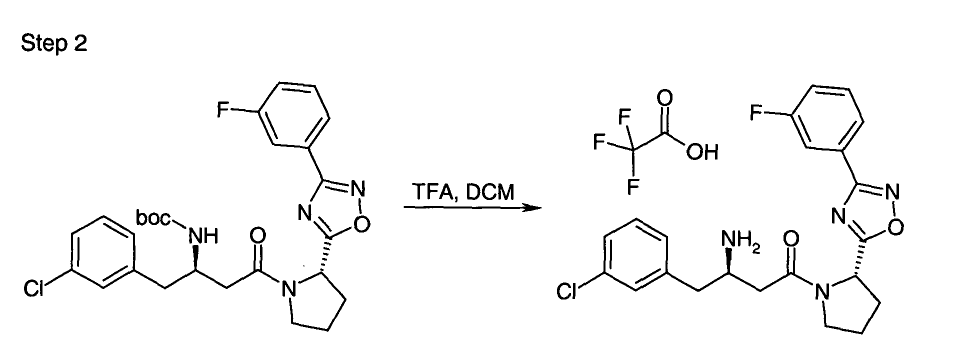

### (R)-3-Amino-4-(3-chloro-phenyl)-1-{(S)-2-[3-(3-fluoro-phenyl)-[1,2,4]oxadiazol-5-yl]-pyrrolidin-1-yl}-butan-1-one trifluoroacetic acid salt

The Boc-protected compound from step 1 is dissolved in 1.00 mL of dichloromethane and is cooled to 0°C. Then 0.50 mL of trifluoroacetic acid is added and the reaction mixture is allowed to warm to room temperature. After 1 h the solvents are removed and the crude product is purified by preparative LCMS to yield the title compound.

LCMS (I) rt 2.46, m/z 429 (M+H)⁺, 492 (M+Na+MeCN)⁺.

¹H-NMR (300 MHz, DMSO-d₆) δ = 7.91 (s, 3H, NH₃), 7.89-7.55 (m, 3H, aryl-H), 7.5-7.1 (m, 5H, aryl-H), 5.27 (m, 1 H, CH), 3.69 (m, 2H, CH₂), 3.54 (m, 1 H, CH), 2.96-2.85 (m, 2H, CH₂), 2.65 (m, 2H, CH₂), 2.38-2.35 (m, 1H, CH), 2.10 (m, 2H, CH₂).

### Example 12

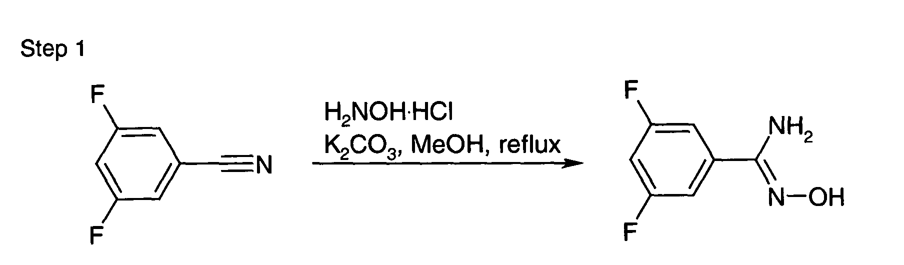

### 3,5-Difluoro-N-hydroxy-benzamidine

100 mg (0.72 mmol, 1.00 eq) of 3,5-difluorobenzonitrile is dissolved in 4.00 mL of methanol and 149 mg (1.08 mmol, 1.50 eq) of potassium carbonate followed by 99.9 mg (1.44 mmol, 2.00 eq) of hydroxylamine hydrochloride are added. The reaction mixture is refluxed for 12 h, filtered and the solvent is evaporated. The crude product is purified by flash chromatography (silica gel, eluent: *c*-hexane:ethyl acetate = 3:2) to yield the title compound.

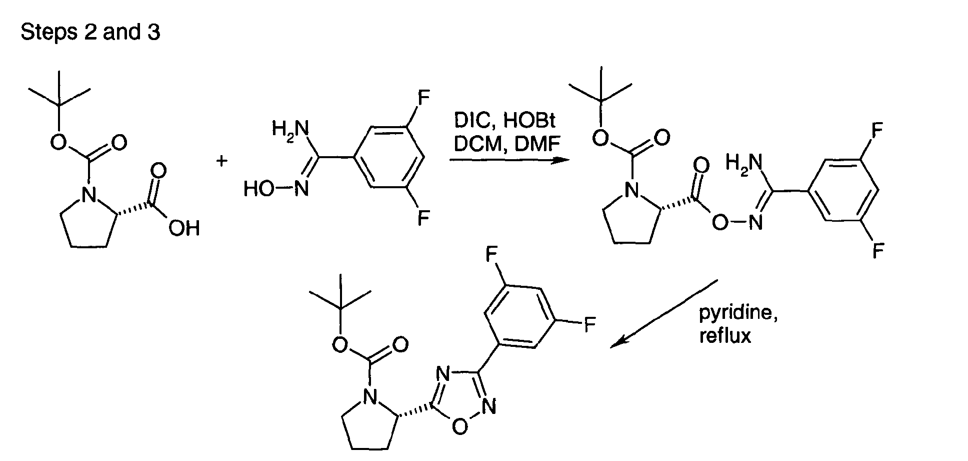

### (S)-2-[3-(3,5-Difluoro-phenyl)-[1,2,4]oxadiazol-5-yl]-pyrrolidine-1-carboxylic acid tert-butyl ester

109 mg (0.63 mmol, 1.00 eq) of 3,5-difluoro-*N*-hydroxy-benzamidine and 150 mg (0.70 mmol, 1.10 eq) of Boc-(*S*)-proline are dissolved in a mixture of 4 mL of dichloromethane and 0.5 mL of *N*,*N*-dimethylformamide. Then 98.9 mg (0.73 mmol, 1.20 eq) 1-hydroxybenzotriazole and 115 µL (92.4 mg, 0.73 mmol, 1.20 eq) *N*,*N*-di-*iso*-propylcarbodimide are added to the reaction mixture. After 12 h stirring at room temperature the reaction mixture is poured into brine, and is further diluted with water. The aqueous mixture is extracted 3 times with ethyl acetate and the combined organic layers are washed with 5 % citric acid solution, saturated sodium bicarbonate solution, brine and dried with sodium sulfate. The solvent is evaporated and the residue is purified by flash chromatography (silica gel, eluent: *c*-hexane:ethyl acetate = 1:1). 203 mg (0.55 mmol) of the *O*-acyl amidoxime is dissolved in 4.00 mL of pyridine and the solution is heated to reflux for 12 h. Then the pyridine is removed in vacuo and the residue is purified via flash chromatography (silica gel, eluent: *c*-hexane:ethyl acetate = 4:1) to yield the title compound.

LCMS (I) rt 3.34, λₘₐₓ = 234 nm (s), 277 nm (w).

¹H-NMR (300 MHz, DMSO-d₆) δ = 7.59 (m, 2H, aryl-H), 7.48 (m, 1H, aryl-H), 5.10 (m, 1H, CH), 3.56-3.23 (m, 2H, CH₂), 2.40-2.38 (m, 1 H, CH), 2.04-1.94 (m, 3H, CH and CH₂), 1.39-1.19 (d, 9H, 3xCH₃).

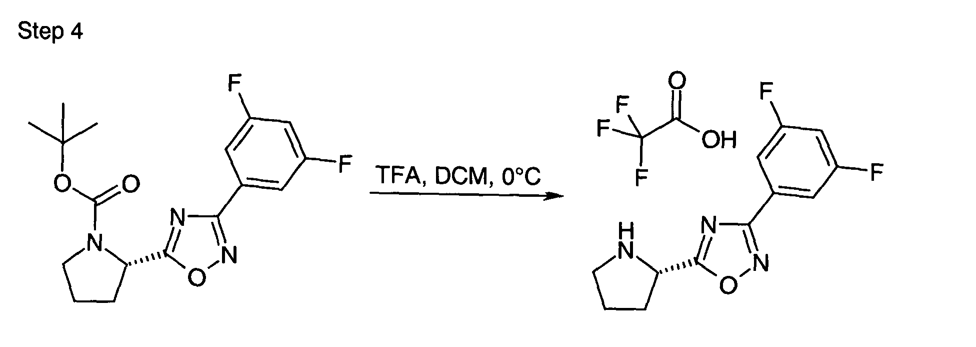

### 3-(3,5-Difluoro-phenyl)-5-(S)-pyrrolidin-2-yl-[1,2,4]oxadiazole trifluoroacetic acetate

46.0 mg (131 µmol, 1.00 eq) of the Boc-protected compound from step 3 is dissolved in 1.00 mL of dichloromethane. The solution is cooled to 0°C and 0.50 mL of trifluoroacedic acid is added. The reaction mixture is allowed to warm to room temperature and after 1 h the solvents are evaporated to yield the crude title compound, which is dried in vacuo and used without further purification in step 5.

LCMS (I) rt 1.85, m/z 252 (M+H)⁺, 293 (M+H+MeCN)⁺.

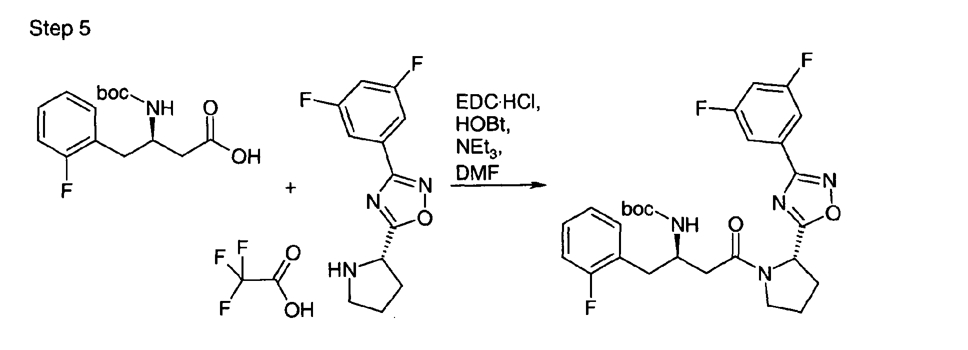

### (R)-[3-{(S)-2-[3-(3,5-Difluoro-phenyl)-[1,2,4]oxadiazol-5-yl]-pyrrolidin-1-yl}-1-(2-fluorobenzyl)-3-oxo-propyl]-carbamic acid tert-butyl ester

14.9 mg (110 mmol, 1.50 eq) of 1-hydroxybenzotriazole, 36.9 µL (26.8 mg, 264 µmol, 3.60 eq) triethylamine and 21.1 mg (110 µmol, 1.50 eq) of 1-ethyl-3-(3-dimethylaminopropyl)carbodiimide hydrochloride are added to a stirred solution of 21.8 mg (73.4 mmol, 1.00 eq) of Boc-(*R*)-3-amino-4-(2-fluoro-phenyl)-butyric acid in 1.00 mL of *N*,*N-*dimethylformamide. The reaction mixture is stirred for 15 min., after which a solution of 26.8 mg (73.4 mmol, 1.00 eq) of the crude deprotected pyrrolidine compound from step 4 in 1.00 mL of *N*,*N*-dimethylformamide is added. The resulting solution is stirred for 12 h, then poured into brine and diluted with water. The aqueous mixture is extracted 3 times with ethyl acetate and the combined organic layers are washed with 5 % citric acid solution, saturated sodium bicarbonate solution, brine and are dried with sodium sulfate. The solvent is evaporated and the residue is subjected to flash chromatography (silica gel, eluent: *c*-hexan:ethyl acetate = 3:2) to yield the title compound.
LCMS (I) rt 3.48, m/z 431 (M+H-Boc)⁺.

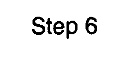

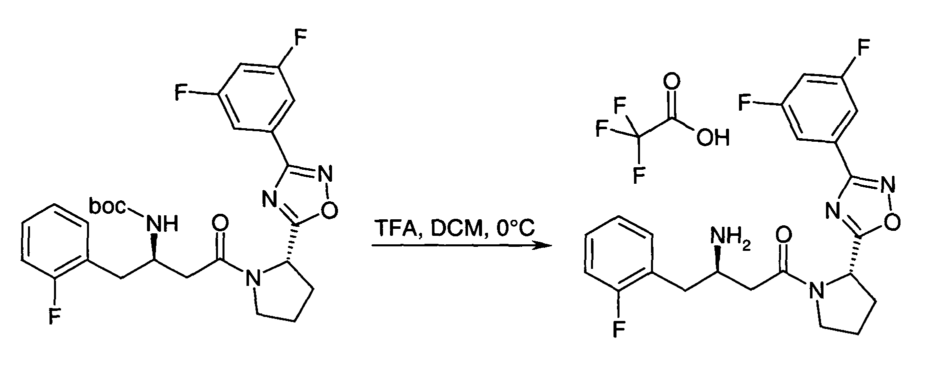

### (R)-3-Amino-1-{(S)-2-[3-(3,5-difluoro-phenyl)-[1,2,4]oxadiazol-5-yl)-pyrrolidin-1-yl}-4-(2-fluoro-phenyl)-butan-1-one trifluoracetic acid salt

42.0 mg (79.2 µmol, 1.00 eq) of the Boc-protected compound from step 5 is dissolved in 1.00 mL of dichloromethane. The solution is cooled to 0°C and 0.50 mL of trifluoroacetic acid is added. The reaction mixture is allowed to warm to room temperature and after 30 min the solvent is evaporated to yield the crude title compound, which is purified by preparative LCMS.

LCMS (I) rt 2.58, 453 (M+Na)⁺, 472 (M+H+MeCN)⁺.

¹H-NMR (300 MHz, DMSO-d₆) δ = 8.1-7.8 (s, 3H, NH₃), 7.6-7.4 (m, 3H, aryl-H), 7.35-7.2 (m, 2H, aryl-H), 7.10-7.17 (m, 2H, aryl-H), 5.26 (m, 1 H, CH), 3.75-3.60 (m, 2H, CH₂), 3.50 (m, 1 H, CH), 2.97 (m, 2H, CH₂), 2.64 (m, 2H, CH₂), 2.35 (m, 1 H, CH), 2.08 (m, 3H, CH and CH₂).

### Example 13

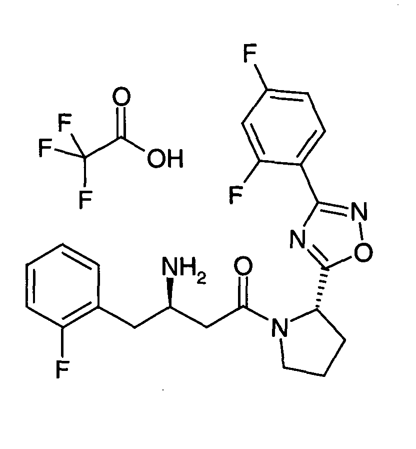

### (R)-3-Amino-1-{(S)-2-[3-(2,4-difluoro-phenyl)-[1,2,4]oxadiazol-5-yl)-pyrrolidin-1-yl}-4-(2-fluoro-phenyl)-butan-1-one trifluoracetic acid salt

The title compound was prepared according to steps 1-6 in example 12.

LCMS (I) rt 2.49, 453 (M+Na)⁺, 472 (M+H+MeCN)⁺.

¹H-NMR (300 MHz, DMSO-d₆) δ = 8.03-7.89 (m, 4H, NH₃, aryl-H), 7.45 (m, 1H, aryl-H), 7.32-7.27 (m, 3H, aryl-H), 7.23-7.09 (m, 2H, aryl-H), 5.27 (m, 1 H, CH), 3.67 (m, 2H, CH₂), 3.51 (m, 1H, CH), 2.97 (m, 2H, CH₂), 2.65 (m, 2H, CH₂), 2.35 (m, 1H, CH), 1.96 (m, 3H, CH and CH₂).

### Example 14

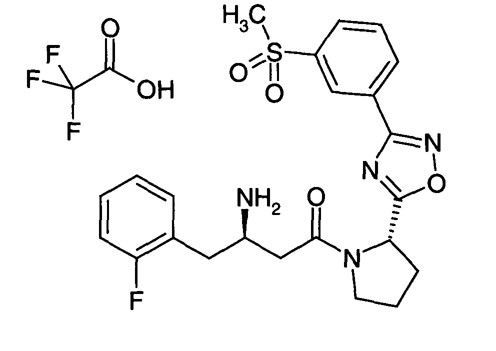

### (R)-3-Amino-1-{(S)-2-[3-(3-methanesulfonyl-phenyl)-[1,2,4]oxadiazol-5-yl)-pyrrolidin-1-yl}-4-(2-fluoro-phenyl)-butan-1-one trifluoracetic acid salt

The title compound was prepared according to steps 1-6 in example 12.

LCMS (I) rt 2.31, 473 (M+H)⁺, 495 (M+Na)⁺.

¹H-NMR (300 MHz, DMSO-d₆) δ = 8.37 (s, 1H, aryl-H), 8.24 (d, 1H, aryl-H), 8.11 (d, 1H, aryl-H), 8.0-7.9 (s, 3H, NH₃), 7.81 (dd, 1 H, aryl-H), 7.30 (m, 2H, aryl-H), 7.14 (m, 2H, aryl-H), 5.27 (m, 1H, CH), 3.67 (m, 2H, CH₂), 3.52 (m, 1H, CH), 3.26 (s, 3H, CH₃), 2.96 (m, 2H, CH₂), 2.66 (m, 2H, CH₂), 2.35 (m, 1H, CH), 2.09 (m, 3H, CH and CH₂).

### Example 15

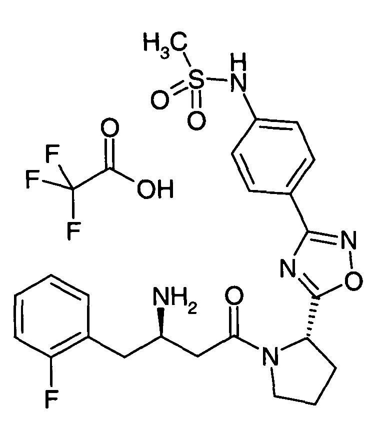

### N-[4-(5-{(S)-1-[(R)-3-Amino-4-(2-fluoro-phenyl)-butyryl]-pyrrolidin-2-yl}-[1,2,4]oxadiazol-3-yl)-phenyl]-methanesulfonamide trifluoroacetic acid salt

The title compound was prepared according to steps 1-6 in example 12.

LCMS (I) rt 2.15, 488 (M+H)⁺, 529 (M+MeCN)⁺.

¹H-NMR (300 MHz, DMSO-d₆) δ = 10.1 (s, 1H, NH), 7.94-7.84 (m, 4H, NH₂ and 2x aryl-H), 7.34-7.28 (m, 4H, aryl-H), 7.16-7.09 (m, 2H, aryl-H), 5.22 (m, 1 H, CH), 3.64-3.48 (m, 3H, CH and CH₂), 3.06 (s, 3H, CH₃), 2.99-2.94 (m, 2H, CH₂), 2.64 (m, 2H, CH₂), 2.34 (m, 1H, CH), 2.06 (m, 2H, CH and CH₂).

### Example 16

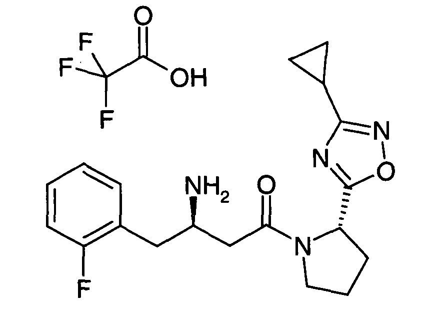

### (R)-3-Amino-1-[(S)-2-(3-cyclopropyl-[1,2,4]oxadiazol-5-yl)-pyrrolidin-1-yl]-4-(2-fluorophenyl)-butan-1-one trifluoracetic acid salt

The title compound was prepared according to steps 1-6 in example 12.

LCMS (I) rt 2.12, 359 (M+H)⁺, 400 (M+MeCN)⁺.

¹H-NMR (300 MHz, DMSO-d₆) δ = 7.94 (s, 3H, NH₃), 7.31 (m, 2H, aryl-H), 7.15 (m, 2H, aryl-H), 5.10 (m, 1 H, CH), 3.66 (m, 2H, CH₂), 3.45 (m, 1 H, CH), 2.94 (m, 2H, CH₂), 2.62 (m, 2H, CH₂), 2.27 (m, 1 H, CH), 2.04-1.95 (m, 4H, 2xCH and CH₂), 1.02 (m, 2H, *c*-propyl-CH₂), 0.82 (m, 2H, *c*-propyl-CH₂)-

### Example 17

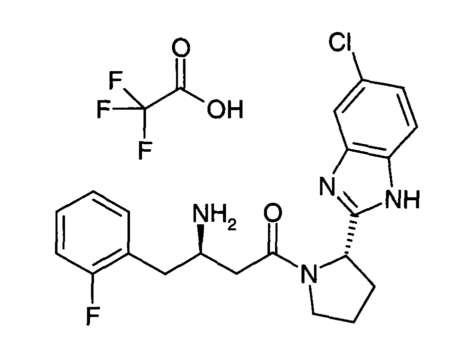

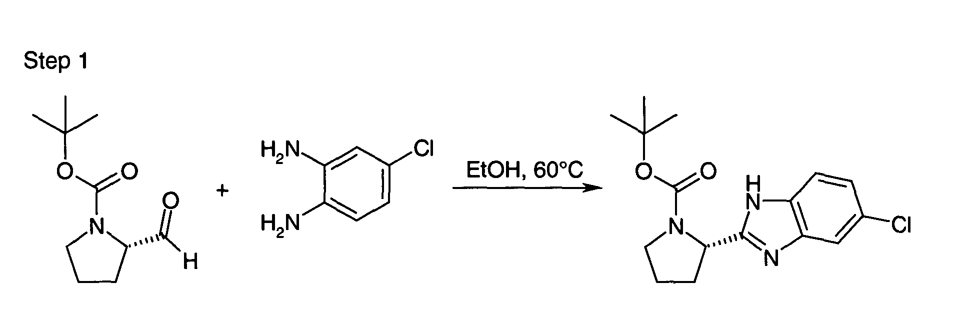

### tert-Butoxy-[(S)-2-(5-chloro-1H-benzoimidazol-2-yl)-pyrrolidin-1-yl]-methanol

100 mg (0.50 mmol, 1.00 eq) of *N*-Boc-(*S*)-prolinal and 72.0 mg (0.50 mmol, 1.00 eq) of 2-amino-4-chloro-aniline are dissolved in ethanol and heated under an air atmosphere to 60°C for 2 days. The resulting solution is evaporated to dryness and the crude product is purifed by flash chromatography (silica gel, eluent: dichloromethane:methanol = 98:2) to yield the title compound.

LCMS (I) rt 2.09, 322 (M+H)⁺.

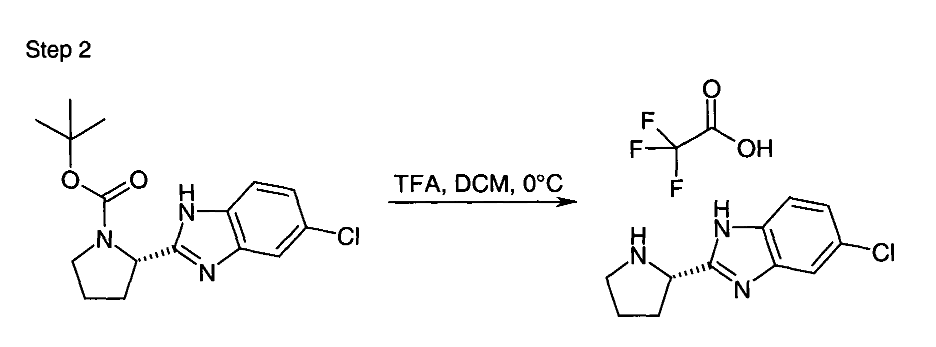

### 5-Chloro-(S)-2-pyrrolidin-2-yl-1H-benzoimidazole trifluoroacetic acid salt

36.4 mg (113 mmol, 1.00 eq) of the Boc protected compound from step 1 is dissolved in 1.00 mL of dichloromethane and at 0°C 0.50 mL trifluoroacetic acid is added to the mixture. The solution is allowed to warm to room temperature and after 1 h the solvents are evaporated to yield the title compound. The crude product is used without further purification in step 3.

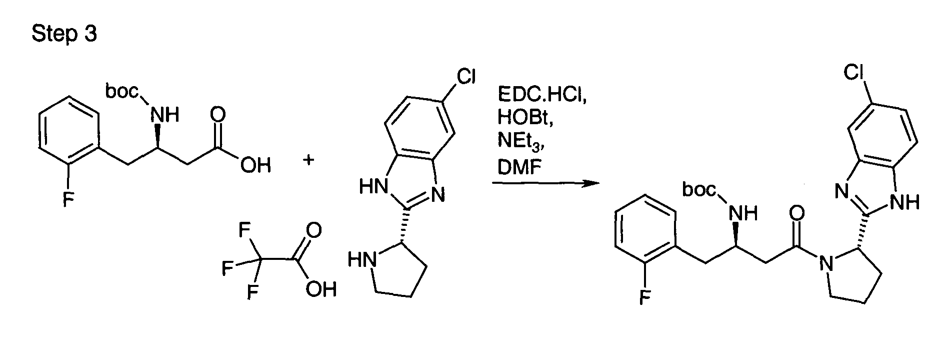

### (R)-[3-[(S)-2-(5-Chloro-1H-benzoimidazol-2-yl)-pyrrolidin-1-yl]-1-(2-fluoro-benzyl)-3-oxo-propyl]- carbamic acid tert-butyl ester

19.1 mg (141 mmol, 1.50 eq) of 1-hydroxybenzotriazole, 47.3 µL (34.3 mg, 339 µmol, 3.60 eq) triethylamine and 27.1 mg (141 µmol, 1.50 eq) of 1-ethyl-3-(3-dimethylaminopropyl)carbodiimide hydrochloride are added to a stirred solution of 28.0 mg (94.0 mmol, 1.00 eq) of Boc-(*R*)-3-amino-4-(2-fluoro-phenyl)-butyric acid in 1.00 mL of *N*,*N*-dimethylformamide. The reaction mixture is stirred for 15 min. at room temperature. Then a solution of 37.9 mg (113 mmol, 1.20 eq) of the crude deprotected pyrrolidine compound from step 2 in 1.00 mL of *N,N*-dimethylformamide is added. The resulting solution is stirred for 12 h, then poured into brine and diluted with water. The aqueous mixture is extracted 3 times with ethyl acetate and the combined organic layers are washed with 5 % citric acid solution, saturated sodium bicarbonate solution, brine and are dried with sodium sulphate. The solvent is evaporated and the residue is subjected to flash chromatography (silica gel, eluent: *c*-hexan:ethyl acetate = 1:1) to yield the title compound.

LCMS (I) rt 2.56, 501 (M+H)⁺.

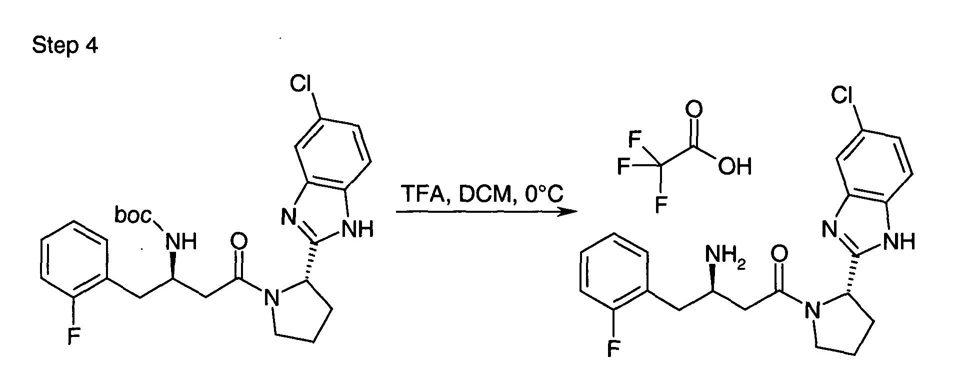

### (R)-3-Amino-1-[(S)-2-(5-chloro-1H-benzoimidazol-2-yl)-pyrrolidin-1-yl]-4-(2-fluorophenyl)-butan-1-one trifluoroacetic acid salt

The purified Boc-protected compound from step 3 is dissolved in 1.00 mL of dichloromethane. The solution is cooled to 0°C and 0.50 mL of trifluoroacetic acid is added. The reaction mixture is allowed to warm to room temperature and after 30 min. the solvent is evaporated to yield the crude title compound, which is purified by preparative LCMS.

LCMS (I) rt 1.91, 401 (M+H)⁺.

¹H-NMR (300 MHz, DMSO-d₆) δ = 7.92 (m, 3H, NH), 7.59-7.51 (m, 2H, aryl-H), 7.33-7.03 (m, 5H, aryl-H), 5.18 (m, 1H, CH), 3.60-3.50 (m, 3H, CH, CH₂) [overlayed by water peak], 3.00-2.90 (m, 2H, CH₂), 2.63 (m, 2H, CH₂), 2.31-2.30 (m, 1H, CH), 2.08-2.02 (m, 3H, CH, CH₂).

### ASSAYS

Inhibition of DPP-IV peptidase activity was monitored with a continuous fluorimetric assay. This assay is based on the cleavage of the substrate Gly-Pro-AMC (Bachem) by DPP-IV, releasing free AMC. The assay is carried out in 96-well microtiterplates. In a total volume of 100 µl, compounds are preincubated with 50 pM DPP-IV employing a buffer containing 10mM Hepes, 150mM NaCl, 0.005% Tween 20 (pH 7.4). The reaction is started by the addition of 16 µM substrate and the fluorescence of liberated AMC is detected for 10 minutes at 25 °C with a fluorescence reader (BMG-Fluostar; BMG-Technologies) using an excitation wavelength of 370 nm and an emission wavelength of 450 nm. The final concentration of DMSO is 1 %. The inhibitory potential of the compounds were determined. DPP-IV activity assays were carried out with human and porcine DPP-IV (see below); both enzymes showed comparable activities.

Soluble human DPP-IV lacking the transmembrane anchor (Gly31-Pro766) was expressed in a recombinant YEAST-strain as Pre-Pro-alpha-mating fusion. The secreted product (rhuDPP-IV-Gly31-Pro766) was purified from fermentation broth (>90% purity).

The examples 1-17 show a % inhibition of about 2 µM or less.

## Claims

1. A compound of the formula (I) 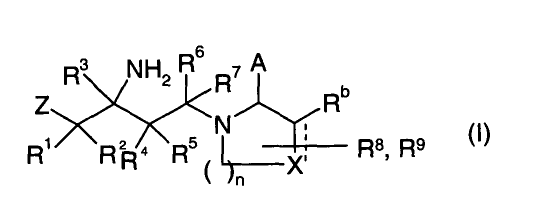 or a pharmaceutically acceptable salt thereof, wherein a dotted line indicates an optionally present double bond and wherein
Z is selected from the group consisting of phenyl; naphthyl; indenyl; C₃₋₇ cycloalkyl; indanyl; tetralinyl; decalinyl; heterocycle; and heterobicycle, wherein Z is optionally substituted with one or more R¹⁰, wherein R¹⁰ is independently selected from the group consisting of halogen; CN; OH; NH₂; oxo (=O), where the ring is at least partially saturated; R¹¹; and R¹²;
R¹¹ is selected from the group consisting of C₁₋₆ alkyl; O-C₁₋₆ alkyl; and S-C₁₋₆ alkyl, wherein R¹¹ is optionally interrupted by oxygen and wherein R¹¹ is optionally substituted with one or more halogen independently selected from the group consisting of F; and Cl;
R¹² is selected from the group consisting of phenyl; heterocycle; and C₃₋₇ cycloalkyl, wherein R¹² is optionally substituted with one or more R¹³, wherein R¹³ is independently selected from the group consisting of halogen; CN; OH; NH₂; oxo (=O), where the ring is at least partially saturated; C₁₋₆ alkyl; O-C₁₋₆ alkyl; and S-C₁₋₆ alkyl;
R¹, R⁴ are independently selected from the group consisting of H; F; OH; and R¹⁴;
R², R⁵, R⁶, R⁷ are independently selected from the group consisting of H; F; and R¹⁵;
R¹⁴ is independently selected from the group consisting of C₁₋₆ alkyl; O-C₁₋₆ alkyl; N(R^{14a})-C₁₋₆ alkyl; S-C₁₋₆ alkyl; C₃₋₇ cycloalkyl; O-C₃₋₇ cycloalkyl; N(R^{14a})-C₃₋₇ cycloalkyl; S-C₃₋₇ cycloalkyl; -C₁₋₆ alkyl-C₃₋₇ cycloalkyl; O-C₁₋₆ alkyl-C₃₋₇ cycloalkyl; N(R^{14a})-C₁₋₆ alkyl-C₃₋₇ cycloalkyl; S-C₁₋₆ alkyl-C₃₋₇ cycloalkyl; heterocycle; O-heterocycle; N(R^{14a})- heterocycle; S-heterocycle; C₁₋₆ alkyl-heterocycle; O-C₁₋₆ alkyl-heterocycle; N(R^{14a})-C₁₋₆ alkyl-heterocycle; S-C₁₋₆ alkyl-heterocycle; wherein R¹⁴ is optionally substituted with one or more halogen independently selected from the group consisting of F; and Cl;
R^{14a} is selected from the group consisting of H; and C₁₋₆ alkyl;
Optionally R⁶ is selected from the group consisting of -C(R^{6a}R^{6b})-O-C₁₋₆ alkyl; -C(R^{6a}R^{6b})-O-C₃₋₇ cycloalkyl; -C(R^{6a}R^{6b})-S-C₁₋₆ alkyl; -C(R^{6a}R^{6b})-S-C₃₋₇ cycloalkyl; -C(R^{6a}R^{6b})-N(R^{6c})-C₁₋₆ alkyl; and -C(R^{6a}R^{6b})-N(R^{6c})-C₃₋₇ cycloalkyl, wherein each C₁₋₆ alkyl and C₃₋₇ cycloalkyl is optionally substituted with one or more R^{6d}, wherein R^{6d} is independently selected from the group consisting of halogen; C₁₋₆ alkyl; and C₃₋₇ cycloalkyl;
R^{6a}, R^{6b}, R^{6c} are independently selected from the group consisting of H; and C₁₋₆ alkyl;
R¹⁵ is independently selected from the group consisting of C₁₋₆ alkyl; C₃₋₇ cycloalkyl; and -C₁₋₆ alkyl-C₃₋₇ cycloalkyl, wherein R¹⁵ is optionally substituted with one or more R^{15a}, wherein R^{15a} is independently selected from the group consisting of F; Cl; and OH;
R³ is selected from the group consisting of H; and C₁₋₆ alkyl;
Optionally one or more pairs of R¹, R², R³, R⁴, R⁵, R⁶, R^{6a}, R^{6b}, R⁷ independently selected from the group consisting of R¹/R²; R²/R³; R³/R⁴; R⁴/R⁵; R⁵/R⁶; R^{6a}/R^{6b} and R⁶/R⁷ form a C₃₋₇ cycloalkyl ring, which is optionally substituted with one or more of R^{15b}, wherein R^{15b} is independently selected from the group consisting of F; Cl; and OH;
Optionally R⁶, R⁷ jointly form an oxo (=O);
n is 0, 1, 2 or 3;
X is selected from the group consisting of -C(R¹⁶R^{c})-; -C(R^{a})=CR^{c}-; -C(R¹⁶R^{a})-CR^{c}=, -C(R¹⁶R^{a})-O-; -C(R¹⁶R^{a})-S-; -C(R¹⁶R^{a})-S(O)-; -C(R¹⁶R^{a})-S(O)₂-; -C(R¹⁶R^{a})-NR^{c}-; and -C(R¹⁶R^{a})-CR¹⁷R^{c}-;
R⁸ is selected from the group consisting of H; F; OH; and C₁₋₆ alkyl, optionally substituted with one or more halogen selected from the group consisting of F; and Cl;
R⁹, R¹⁶, R¹⁷ are independently selected from the group consisting of H; F; and C₁₋₆ alkyl, optionally substituted with one or more halogen selected from the group consisting of F; and Cl;
R^{a}, R^{c} are independently selected from the group consisting of H; F; Cl; and CN;
R^{b}, A are independently selected from the group consisting of H; F; Cl; CN; and A¹, provided that
(a) one of R^{b}, A is A¹; and
(b) when A is A¹ and n is 1 and R⁶, R⁷ jointly form an oxo (=O), X is other than -C(R¹⁶R^{a})-O-; -C(R¹⁶R^{a})-NR^{c}-; or -C(R¹⁶R^{a})-CR¹⁷R^{c}-; and
(c) when A is A¹ and n is 2 and R⁶, R⁷ jointly form an oxo (=O), X is other than -C(R¹⁶R^{c})-;
Optionally R^{c} is selected from the group consisting of -O-C₁₋₆ alkyl; -O-C₃₋₇ cycloalkyl; -S-C₁₋₆ alkyl; -S-C₃₋₇ cycloalkyl; -N(R¹⁸)-C₁₋₆ alkyl; and -N(R¹⁸)-C₃₋₇ cycloalkyl, wherein each C₁₋₆ alkyl and C₃₋₇ cycloalkyl is optionally substituted with one or more R^{18a}, wherein R^{18a} is independently selected from the group consisting of halogen; C₁₋₆ alkyl; and C₃₋₇ cycloalkyl, provided that n is 1;
R¹⁸ is independently selected from the group consisting of H; C₁₋₆ alkyl;
Optionally a pair of R^{a}, R^{b}, R^{c} selected from the group consisting of R^{a}/R^{c}; and R^{b}/R^{c} forms a ring Z¹; provided that R^{b}/R^{c} form a ring other than
(a) pyrimidine when R⁶, R⁷ jointly form an oxo (=O) and n is 1 and X is -C(R¹⁶R^{a})-C(R^{c})=;
(b) a ring selected from the group consisting of 1,2-diazole; and 1,2,4-triazole when R⁶, R⁷ jointly form an oxo (=O) and n is 1 and X is -C(R¹⁶R^{a})-N(R^{c})-;
Z¹ is selected from the group consisting of Z²; and Z³;
Z² is selected from the group consisting of phenyl; naphthyl; and indenyl; wherein Z² is optionally substituted with one or more R¹⁹; wherein R¹⁹ is independently selected from the group consisting of halogen; CN; COOR²⁰; OR²⁰; C(O)N(R²⁰R^{20a}); S(O)₂N(R²⁰R^{20a}); C₁₋₆ alkyl; O-C₁₋₆ alkyl; S-C₁₋₆ alkyl; COO-C₁₋₆ alkyl; OC(O)-C₁₋₆ alkyl; C(O)N(R²⁰)-C₁₋₆ alkyl; S(O)₂N(R²⁰)-C₁₋₆ alkyl; S(O)N(R²⁰)-C₁₋₆ alkyl; S(O)₂-C₁₋₆ alkyl; S(O)-C₁₋₆ alkyl; N(R²⁰)S(O)₂-C₁₋₆ alkyl; and N(R²⁰)S(O)-C₁₋₆ alkyl; wherein each C₁₋₆ alkyl is optionally substituted with one more halogen selected from the group consisting of F; and Cl;
Z³ is selected from the group consisting of C₃₋₇ cycloalkyl; indanyl; tetralinyl; decalinyl; heterocycle; and heterobicycle; wherein Z³ is optionally substituted with one or more R²¹, wherein R²¹ is independently selected from the group consisting of halogen; CN; OR²²; oxo (=O), where the ring is at least partially saturated; N(R²²R^{22a}); COOR²²; C(O)N(R²²R^{22a}); S(O)₂N(R²²R^{22a}); S(O)N(R²²R^{22a}); C₁₋₆ alkyl; O-C₁₋₆ alkyl; S-C₁₋₆ alkyl; N(R²²)-C₁₋₆ alkyl; COO-C₁₋₆ alkyl; OC(O)- C₁₋₆ alkyl; C(O)N(R²²)- C₁₋₆ alkyl; N(R²²)-C(O)-C₁₋₆ alkyl; S(O)₂N(R²²)-C₁₋₆ alkyl; S(O)N(R²²)-C₁₋₆ alkyl; S(O)₂-C₁₋₆ alkyl; S(O)-C₁₋₆ alkyl; N(R²²)S(O)₂-C₁₋₆ alkyl; and N(R²²)S(O)-C₁₋₆ alkyl; wherein each C₁₋₆ alkyl is optionally substituted with one more halogen selected from the group consisting of F; and Cl;
Optionally R²¹ is C(O)R²², provided that C(O)R²² is bound to a nitrogen, which is a ring atom of a heterocycle or heterobicycle;
R²⁰, R^{20a}, R²², R^{22a} are independently selected from the group consisting of H; C₁₋₆ alkyl; C₃₋₇ cycloalkyl; and -C₁₋₆ alkyl-C₃₋₇ cycloalkyl;
A¹ is selected from the group consisting of phenyl; heterocycle; heterobicycle; C₃₋₇ cycloalkyl, wherein A¹ is substituted with R²³ and wherein phenyl is optionally substituted with one R²⁴ and wherein heterocycle; heterobicycle; C₃₋₇ cycloalkyl are optionally substituted with one R²⁵;
R²⁴ is selected from the group consisting of halogen; CN; COOR²⁶; OC(O)R²⁶; OR²⁶; -C₁₋₆alkyl-OR²⁶; SR²⁶; C(O)N(R²⁶R²⁷); S(O)₂N(R²⁶R²⁷); S(O)N(R²⁶R²⁷); C₁₋₆ alkyl; N(R²⁶)S(O)₂R²⁷; and N(R²⁶)S(O)R²⁷; wherein each C₁₋₆ alkyl is optionally substituted with one more halogen selected from the group consisting of F; and Cl;
R²⁵ is selected from the group consisting of halogen; CN; OR²⁶; -C₁₋₆alkyl-OR²⁶ SR²⁶; oxo (=O), where the ring is at least partially saturated; N(R²⁶R²⁷); COOR²⁶; OC(O)R²⁶; C(O)N(R²⁶R²⁷); S(O)₂N(R²⁶R²⁷); S(O)N(R²⁶R²⁷); C₁₋₆ alkyl; N(R²⁶)C(O)R²⁷; S(O)₂R²⁶; S(O)R²⁶; N(R²⁶)S(O)₂R²⁷; and N(R²⁶)S(O)R²⁷; wherein each C₁₋₆ alkyl is optionally substituted with one more halogen selected from the group consisting of F; and Cl;
Optionally R²⁵ is C(O)R²⁶, provided that C(O)R²⁶ is bound to a nitrogen, which is a ring atom of a heterocycle or heterobicycle;
R²⁶, R²⁷ are independently selected from the group consisting of H; C₁₋₆ alkyl; C₃₋₇ cycloalkyl; and -C₁₋₆ alkyl-C₃₋₇ cycloalkyl; wherein each C₁₋₆ alkyl is optionally substituted with one more halogen selected from the group consisting of F; and Cl;
R²³ is selected from the group consisting of F; Cl; oxo (=O), where the ring is at least partially saturated; OR^{23a}; N(R^{23a}R^{23b}); C₁₋₆ alkyl, optionally substituted with one or more R²⁸; and -(C(R²⁹R^{29a}))ₘ-W-(C(R³⁰R^{30a}))ₒ-T;
R²⁸ is selected from the group consisting of halogen; CN; COOR³¹; OC(O)R³¹; OR³¹; SR³¹; C(O)N(R³¹R³²); S(O)₂N(R³¹R³²); S(O)N(R³¹R³²); N(R³¹)S(O)₂R³²; and N(R³¹)S(O)R³²;
R²⁹, R^{29a}, R³⁰, R^{30a} are independently selected from the group consisting of H; F; and R³³;
Optionally one or both pairs of R²⁹, R^{29a}, R³⁰, R^{30a} independently selected from the group consisting of R²⁹/R^{29a}; and R³⁰/R^{30a} form a C₃₋₇ cycloalkyl ring, which is optionally substituted with one or more of R^{33b}, wherein R^{33b} is independently selected from the group consisting of F; Cl; and OH;
R^{23a}, R^{23b}, R³¹, R³² are independently selected from the group consisting of H; C₁₋₆ alkyl; C₃₋₇ cycloalkyl; and -C₁₋₆ alkyl-C₃₋₇ cycloalkyl; wherein each C₁₋₆ alkyl is optionally substituted with one more halogen selected from the group consisting of F; and Cl;
R³³ is selected from the group consisting of C₁₋₆ alkyl; C₃₋₇ cycloalkyl; and -C₁₋₆ alkyl-C₃₋₇ cycloalkyl, wherein R³³ is optionally substituted with one or more R^{33a}, wherein R^{33a} is independently selected from the group consisting of F; Cl; and OH;
m, o are independently selected from the group consisting of 0; 1; 2; and 3;
W is selected from the group consisting of a covalent bond; -O- -S-; -S(O₂)-;-S(O)-; -N(R³⁴)-; -N(R³⁴)C(O)-; -C(O)N(R³⁴)-; -OC(O)-; -C(O)O-; -S(O₂)N(R³⁴)-; -N(R³⁴)S(O)₂-; S(O)N(R³⁴)-; and -N(R³⁴)S(O)-;
R³⁴ is selected from the group consisting of H; C₁₋₆ alkyl; C₃₋₇ cycloalkyl; and -C₁₋₆ alkyl-C₃₋₇ cycloalkyl; wherein each C₁₋₆ alkyl is optionally substituted with one more halogen selected from the group consisting of F; and Cl;
T is selected from the group consisting of H; T¹; and T²;
T¹ is selected from the group consisting of phenyl; naphthyl; and indenyl; wherein T¹ is optionally substituted with one or more R³⁵; wherein R³⁵ is independently selected from the group consisting of halogen; CN; COOR³⁷; OC(O)R³⁷; OR³⁷; -C₁₋₆alkyl-OR³⁷; SR³⁷; S(O)R³⁷; S(O)₂R³⁷; C(O)N(R³⁷R³⁸); S(O)₂N(R³⁷R³⁸); S(O)N(R³⁷R³⁸); C₁₋₆ alkyl; N(R³⁷)S(O)₂R³⁸; and N(R³⁷)S(O)R³⁸; wherein each C₁₋₆ alkyl is optionally substituted with one more halogen selected from the group consisting of F; and Cl;
T² is selected from the group consisting of C₃₋₇ cycloalkyl; indanyl; tetralinyl; decalinyl; heterocycle; and heterobicycle; wherein T² is optionally substituted with one or more R³⁶, wherein R³⁶ is independently selected from the group consisting of halogen; CN; OR³⁷; -C₁₋₆alkyl-OR³⁷ SR³⁷; oxo (=O), where the ring is at least partially saturated; N(R³⁷R³⁸); COOR³⁷; OC(O)R³⁸; C(O)N(R³⁷R³⁸); S(O)₂N(R³⁷R³⁸); S(O)N(R³⁷R³⁸); C₁₋₆ alkyl; N(R³⁷)C(O)R³⁸; S(O)₂R³⁷; S(O)R³⁸; N(R³⁷)S(O)₂R³⁸; and N(R³⁷)S(O)R³⁸; wherein each C₁₋₆ alkyl is optionally substituted with one more halogen selected from the group consisting of F; and Cl;
Optionally R³⁶ is C(O)R³⁷, provided that C(O)R³⁷ is bound to a nitrogen, which is a ring atom of a heterocycle or heterobicycle;
R³⁷, R³⁸ are independently selected from the group consisting of H; C₁₋₆ alkyl; C₃₋₇ cycloalkyl; and -C₁₋₆ alkyl-C₃-₇ cycloalkyl; wherein each C₁₋₆ alkyl is optionally substituted with one more halogen selected from the group consisting of F; and Cl.

2. A compound according to claim 1 of formula (Ia) 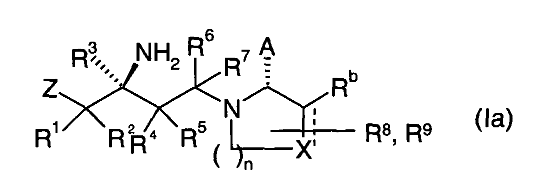 or a pharmaceutically acceptable salt thereof, wherein Z, R¹⁻⁹, n, A, X and R^{b} have the meaning as indicated in claim 1.

3. A compound according to claim 1 or 2, wherein Z is selected from the group consisting of phenyl; and heterocycle; and wherein Z is optionally substituted with up to 2 R¹⁰, which are the same or different.

4. A compound according to any one of the preceding claims, wherein R¹⁰ is selected from the group consisting of F; Cl; CN; and C₁₋₆ alkyl.

5. A compound according to any one of the preceding claims, wherein R¹, R², R⁴, R⁵ are independently selected from the group consisting of H; F; and C₁₋₆ alkyl, optionally substituted with one or more F.

6. A compound according to any one of the preceding claims, wherein R³ is H.

7. A compound according to any one of the preceding claims, wherein R⁶ and R⁷ jointly form an oxo (=O).

8. A compound according to any one of the preceding claims, wherein R⁶ and R⁷ are independently selected from the group consisting of H; and C₁₋₆ alkyl, optionally substituted with one or more F.

9. A compound according to any one of the preceding claims, wherein n is 0 and X is -CHR^{a}-CHR^{b}-;

10. A compound according to any one of the preceding claims, wherein n is 1 and X is -CHR^{c}-.

11. A compound according to any one of the preceding claims, wherein n is 1 and X is -C(R^{a})=CR^{c}- and R^{a}/R^{c} forms a ring Z¹.

12. A compound according to any one of the preceding claims, wherein n is 1 and X is -CH(R^{a})-CR^{c}= and R^{a}/R^{c} forms a phenyl.

13. A compound according to any one of the preceding claims, wherein Z¹ is phenyl.

14. A compound according to any one of the preceding claims, wherein R⁸, R⁹ are H.

15. A compound according to any one of the preceding claims, wherein A is A¹.

16. A compound according to any one of the preceding claims, wherein R^{a}, R^{b}, R^{c} are H.

17. A compound according to any one of the preceding claims, wherein A¹ is heterocycle.

18. A compound according to any one of the preceding claims, wherein A¹ is selected from the group consisting of 1,2,4-oxadiazole; 1,2,4-triazole; 1,2,3-triazole; 1,2-diazole; and benzimidazole; wherein A¹ is substituted with R²³ and optionally substituted with R²⁵_{.}

19. A compound according to any one of the preceding claims, wherein R²³ is -(C(R²⁹R^{29a}))ₘ-W-(C(R³⁰R^{30a}))ₒ-T.

20. A compound according to any one of the preceding claims, wherein R²⁵ is Cl.

21. A compound according to any one of the preceding claims, wherein m and o are 0.

22. A compound according to any one of the preceding claims, wherein W is a covalent bond.

23. A compound according to any one of the preceding claims, wherein T is H;

24. A compound according to any one of the preceding claims, wherein T is phenyl, optionally substituted with one or two R³⁵, which are the same or different.

25. A compound according to any one of the preceding claims, wherein T is selected from the group consisting of heterocycle; and C₃₋₇ cycloalkyl; wherein T is optionally substituted with one or two R³⁶, which are the same or different.

26. A compound according to claim 25, wherein heterocycle is selected from the group consisting of pyridine; and azetidine and wherein C₃₋₇ cycloalkyl is selected from the group consisting of cyclopropyl; and cyclobutyl.

27. A compound according to any one of the preceding claims, wherein R³⁵, R³⁶ are independently selected from the group consisting of F; Cl; -S(O)₂-C₁₋₆alkyl; -S(O)₂NH₂; -S(O)₂(C₁₋₆ alkyl)₂; -NH-S(O)₂-C₁₋₆ alkyl; and -N(C₁₋₆ alkyl)-S(O)₂-C₁₋₆ alkyl.

28. A compound according to claim 1 selected from the group consisting of

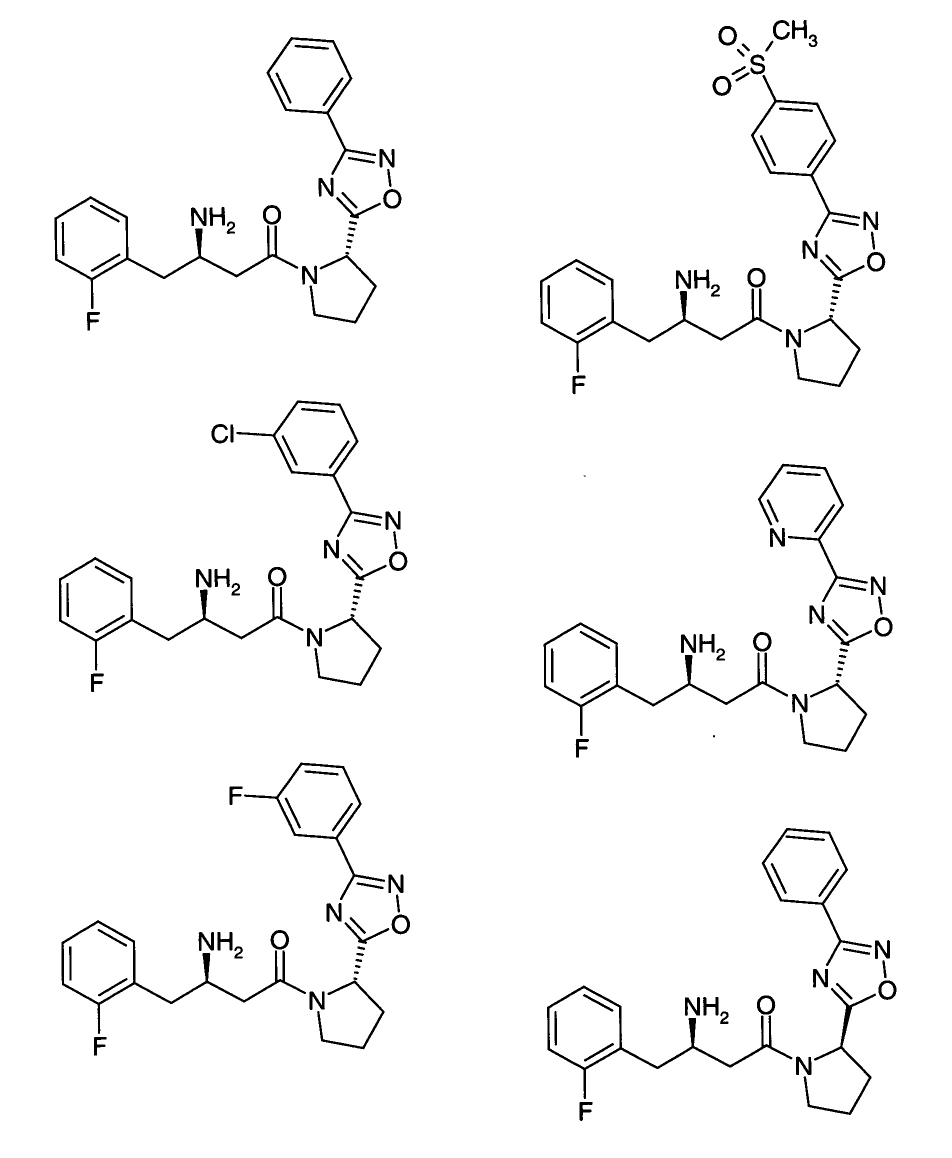

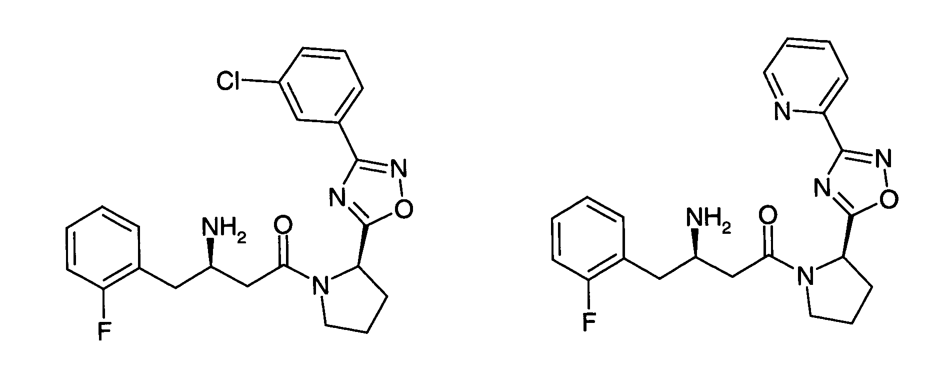

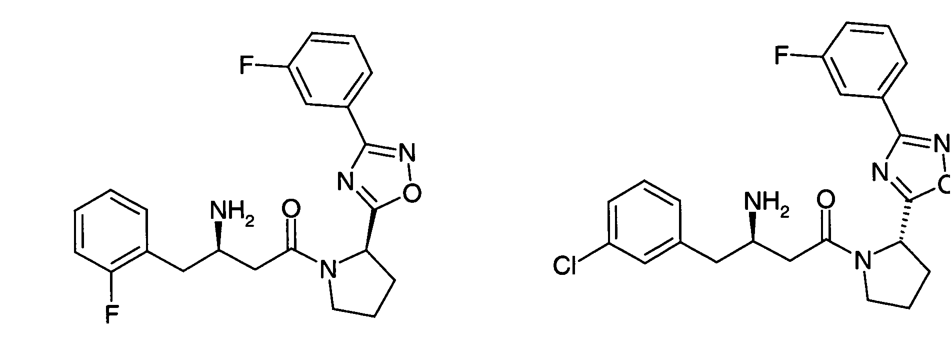

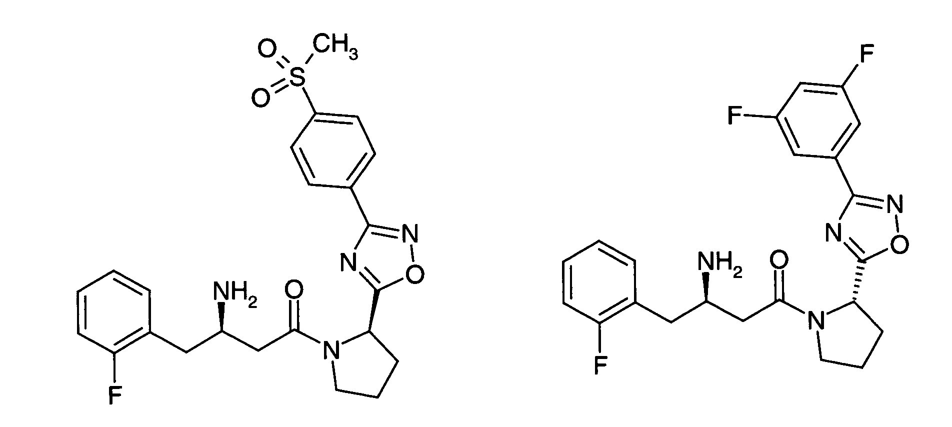

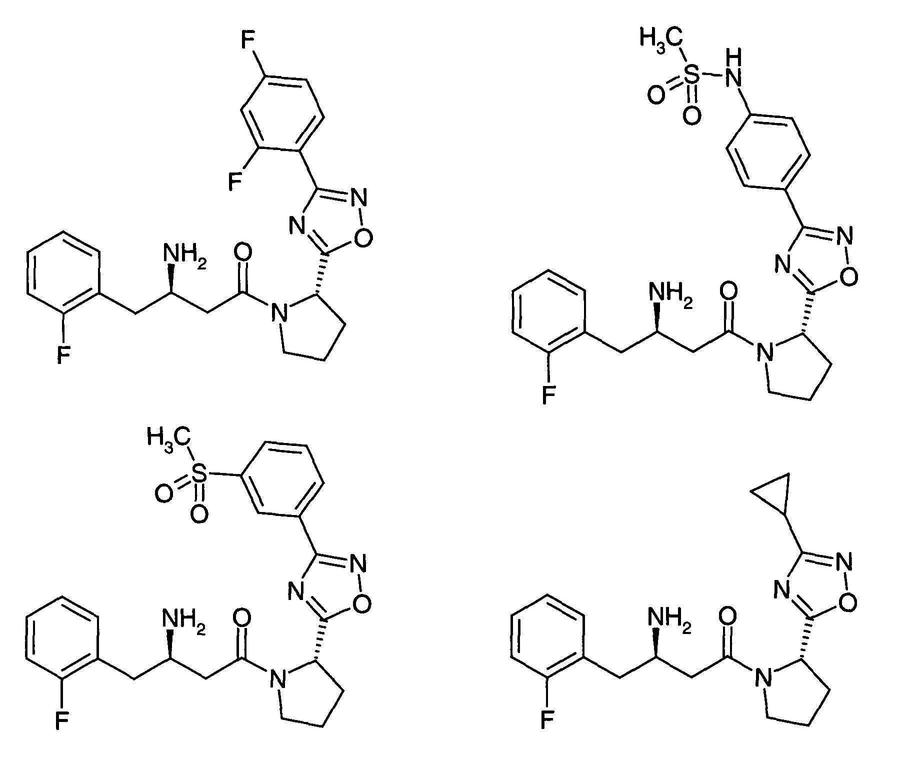

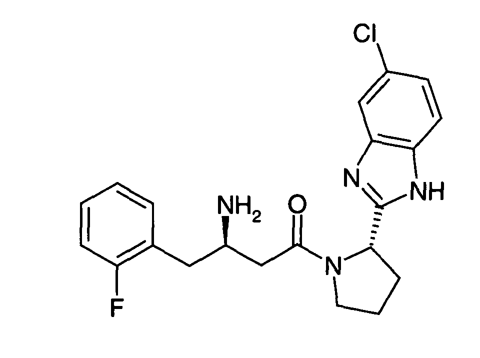

29. A prodrug compound of a compound according to any one of the claims 1 to 28.

30. A pharmaceutical composition comprising a compound or a pharmaceutically acceptable salt thereof according to any one of the claims 1 to 29 together with a pharmaceutically acceptable carrier.

31. A pharmaceutical composition according to claim 30, comprising one or more additional compounds or pharmaceutically acceptable salts thereof selected from the group consisting of another compound according to any one of the claims 1 to 29; another DPP-IV inhibitor; insulin sensitizers; PPAR agonists; biguanides; protein tyrosinephosphatase-IB (PTP-1B) inhibitors; insulin and insulin mimetics; sulfonylureas and other insulin secretagogues; a-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents; HMG-CoA reductase inhibitors; sequestrants; nicotinyl alcohol; nicotinic acid or a salt thereof; PPARa agonists; PPARoly dual agonists; inhibitors of cholesterol absorption; acyl CoA : cholesterol acyltransferase inhibitors; anti-oxidants; PPARo agonists; antiobesity compounds; an ileal bile acid transporter inhibitor; and anti-inflammatory agents.

32. A compound or a pharmaceutically acceptable salt thereof of any one of the claims 1 to 29 for use as a medicament.

33. Use of a compound or a pharmaceutically acceptable salt thereof of any of the claims 1 to 29 for the manufacture of a medicament for the treatment or prophylaxis of non-insulin dependent (Type II) diabetes mellitus; hyperglycemia; obesity; insulin resistance; lipid disorders; dyslipidemia; hyperlipidemia; hypertriglyceridemia; hypercholestrerolemia; low HDL; high LDL; atherosclerosis; growth hormone deficiency; diseases related to the immune response; HIV infection; neutropenia; neuronal disorders; tumor metastasis; benign prostatic hypertrophy; gingivitis; hypertension; osteoporosis; diseases related to sperm motility; low glucose tolerance; insulin resistance; ist sequelae; vascular restenosis; irritable bowel syndrome; inflammatory bowel disease; including Crohn's disease and ulcerative colitis; other inflammatory conditions; pancreatitis; abdominal obesity; neurodegenerative disease; retinopathy; nephropathy; neuropathy; Syndrome X; ovarian hyperandrogenism (polycystic ovarian syndrome; Type n diabetes; or growth hormone deficiency.

34. Use of a compound according to any one of the claims 1 to 29 as a DPP-IV inhibitor.
